# EUROPEAN PATENT APPLICATION

(11) **EP 1 980 317 A1**
(43) Date of publication of application: **15.10.2008**
(21) Application number: 07706647.0
(22) Date of filing: 12.01.2007
(51) Int. Cl.: B01J 20/34, A61L 9/16, A61L 9/22, F24F 1/00

(54) **DEVICE FOR REGAINING DEODORATION FUNCTION AND AIR CONDITIONER EQUIPPED WITH THE DEVICE**

(30) Priority: 19.01.2006 JP 2006010691; 31.07.2006 JP 2006209002
(71) Applicant: DAIKIN INDUSTRIES, LTD., Osaka-shi, Osaka 530-8323 (JP)
(72) Inventor: KAGAWA, Sanaec/o, DAIKIN INDUSTRIES, LTD, Shiga;5258526 (JP); ODA, Yasuhiroc/o Kanaoka Factory, Sakai Plant,, Sakai-shi,i,Osaka;5918511 (JP); YAMASHITA, Tetsuyac/o DAIKIN INDUSTRIES, LTD.,, Kusatsu-shi, Shiga;5258526 (JP); NAGAO, Mitsuhisac/o , DAIKIN INDUSTRIES, LTD., Kusatsu-shi, Shiga;5258526 (JP)
(74) Representative: Goddar, Heinz J.
(86) International application number: PCT/JP2007/050305
(87) International publication number: WO 2007/083573

(57) **Abstract**

It is an object of the present invention to reproduce the deodorization performance of a deodorization functional agent by decomposing odor and harmful gas components adsorbed to the deodorization functional agent not only reliably and simply in a short period of time, but also without generating polluted water. A deodorization function reproducing device (1) is a deodorization function reproducing device for performing a reproducing process of a deodorizing agent (4) that has adsorbed at least one of odor molecules or harmful gas components that are floating in the air. The deodorization function reproducing device (1) includes a casing (2), and an active species generating portion (3). The casing can store the deodorizing agent. The active species generating portion generates active species to decompose or inactivate at least one of the odor molecules or harmful gas components.

## Description

### TECHNICAL FIELD

The present invention relates to a reproducing device of a deodorization functional agent, and an air conditioner including the reproducing device.

### BACKGROUND ART

Since the deodorizing effect of a deodorizing agent lowers after it is used for a certain period of time, it is necessary to replace the deodorizing agent. Consequently, conventionally, devices have been developed that recycle the deodorizing agent by utilizing photocatalyst and reproducing it with natural light, or washing it with water, and so forth.

With the technology of Patent Document 1, by knowing that the water solubility of odor components such as acetic acid, sulfurous acid, ammonia, amine, and the like, which are generated from sensitive material process liquid, is high, and by washing deodorizing agent, which has adsorbed these odor components, with cleaning water, odor components adsorbed by the deodorizing agent are separated and the deodorization performance of the deodorizing agent is recovered.

In addition, with the technology of Patent Document 2, on the flow path of air that is drawn inside a chassis, a photocatalytic deodorizer for decomposing and deodorizing the adsorbed odor components by photoexcitation is arranged, and a light introduction means is provided for photocatalytic deodorizer irradiating with light from the exterior. By having this configuration, odor components adsorbed on the photocatalytic deodorizer are oxidatively decomposed and the deodorizing function is reproduced by actively irradiating with light from the exterior, such as sunlight, interior light, and the like, on the photocatalytic deodorizer, through the light introduction means.
<Patent Document 1> JP-A Publication No. 7-313836
<Patent Document 2> JP-A Publication No. 10-227469

### DISCLOSURE OF THE INVENTION

### PROBLEM TO BE SOLVED BY THE INVENTION

However, washing the deodorizing agent with water is not only troublesome, but sewage treatment also becomes necessary, and drying it also takes time. In addition, in the case of a reproduction done by natural light, reproduction process takes a lot of time because reproduction is difficult for the inner portion of the deodorizing agent where it is difficult for light to reach, and so reproduction thereof is easily affected by the strength of the light and the environment.

It is an object of the present invention to reproduce the deodorization performance of the deodorization functional agent by decomposing odor and harmful gas components adsorbed by the deodorization functional agent, not only reliably in a short period of time and simply, but also without generating polluted water.

### MEANS FOR ACHIEVING THE OBJECT

The deodorization function reproducing device in accordance with a first aspect of the present invention is a deodorization function reproducing device for performing a reproduction process of a deodorization functional agent that has adsorbed at least one of odor molecules or harmful gas components that are floating in the air. The deodorization function reproducing device includes a casing and an active species generating portion. The casing can store the deodorization functional agent. The active species generating portion generates active species to decompose or inactivate at least one of the odor molecules or harmful gas components. Here, the "deodorization functional agent" is not limited to activated charcoal, silica gel, zeolite, and the like, and includes, for example, handkerchiefs, tissue papers, newspapers, corrugated papers, wood, and all of such materials that adsorb odor components.

This deodorization function reproducing device has the deodorization functional agent used in any preferred location disposed inside the casing thereof. In addition, this device generates active species through the active species generating portion. The "active species generating portion" referred here includes, for example, a glow discharger, barrier discharger, streamer discharger, and devices and the like that generate active species by irradiating photocatalyst with ultraviolet light. In addition, "active species" referred here are, for example, high speed electrons, ions, ozone, hydroxy radical, and other such radical species, other excited molecules (excited oxygen molecules, excited nitrogen molecules, excited water molecules) and the like. The active species decompose at least one of the odor molecules or harmful gas components adsorbed by the deodorization functional agent to reproduce the deodorization functional agent.

Therefore, since the deodorization functional agent, that needed to be replaced in every predetermined period of time in the past, can be reused, it is possible to suppress the generation of polluted water and the like from washing it with water, and so forth, and the generation of waste. Consequently, when the reproducing process of the deodorization functional agent is performed, this can reduce burden on the environment. In addition, with a simple operation, reproduction can be done reliably and in a short period of time, and thereby usability can be improved.

The deodorization function reproducing device in accordance with a second aspect of the present invention is the deodorization function reproducing device of the first aspect, wherein a blower is further included therein. The blower generates flow of air inside the casing. The "blower" here includes, for example, a member disposed in the interior of the casing that generates flow of air inside the casing, or a member disposed outside the casing that generates flow of air inside the casing, and the like.

This deodorization function reproducing device further includes a blower for generating flow of air inside the casing. The "blower" referred here is, for example, axial flow type of blower, centrifugal type of blower, diagonal flow type of blower, cross flow type of blower, and the like. Therefore, it is possible to accelerate the exposure of active species to the deodorization functional agent. Consequently, the effect of the reproducing process of the deodorization functional agent can be improved.

The deodorization function reproducing device in accordance with a third aspect of the present invention is the deodorization function reproducing device of the second aspect, wherein a purifying portion is further included for purifying air that passes through a position upwind of the storage portion of the deodorization functional agent and the active species generating portion, in the flow of the air that is generated by the blower.

Here, the purifying portion first purifies the air to be provided to the deodorization functional agent, by being arranged upwind of the active species generating portion and the blower for speeding up the provision of active species to the deodorization functional agent.

As a result, it is possible to perform the reproducing process of the deodorization functional agent using purified air.

The deodorization function reproducing device in accordance with a fourth aspect of the present invention is the deodorization function reproducing device of any of the first to the third aspects, wherein the active species generating portion generates active species for a predetermined period of time.

This deodorization function reproducing device generates active species for a predetermined period of time in the active species generating portion. Therefore, the reproducing process of the deodorization functional agent ends when a predetermined time period is passed. Consequently, after a reproducing process of the deodorization functional agent is started, the reproducing process of the deodorization functional agent can be terminated without a user having to perform an operation for ending it.

The deodorization function reproducing device in accordance with a fifth aspect of the present invention is the deodorization function reproducing device of the fourth aspect, wherein the active species generating portion can adjust the predetermined period of time.

This deodorization function reproducing device can adjust the predetermined period of time for generating the active species in the active species generating portion. The reproducing level of the deodorization functional agent is higher if the time of the reproducing process is longer, and lower if the time of the reproducing process is shorter.

Therefore, a user can adjust the reproducing process level according to the degree of uncleanness of the deodorization functional agent, by setting the time of the reproducing process. Consequently, energy used for the reproducing process can be suppressed because it is possible to prevent the reproducing process from being overly done, for the uncleanness of the deodorization functional agent. In addition, the deodorization functional agent can be reproduced more reliably because an insufficiency in the reproducing process can be resolved, for the uncleanness of the deodorization functional agent.

The deodorization function reproducing device in accordance with a sixth aspect of the present invention is the deodorization function reproducing device of the fourth or the sixth aspect, wherein a processing status detection means is further included. The processing status detection means is able to detect the processing status of the reproducing process. The active species generating portion adjusts the predetermined period of time according to the processing status.

This deodorization function reproducing device further includes a processing status detection means that is able to detect the processing status of the reproducing process. The "processing status detection means" referred here is, for example, a temperature sensor, humidity sensor, gas sensor, and the like.

Therefore, the active species generating portion can adjust the reproducing time according to the processing status detected by the processing status detection means. Consequently, a reproducing process that is suitable for the degree of uncleanness of the deodorization functional agent can be performed automatically. As a result, it is not necessary for a user to set a reproducing process time, and this can reduce operational burden on the user, and a reproducing process can be done easily. In addition, energy used for a reproducing process can be suppressed, because it is possible to suppress a reproducing process from being performed excessively for the uncleanness of the deodorization functional agent. Furthermore, the deodorization functional agent can be reproduced more reliably because an insufficiency in a reproducing process that is insufficient for the degree of uncleanness of the deodorization functional agent can be resolved.

The deodorization function reproducing device in accordance with a seventh aspect of the present invention is the deodorization function reproducing device of the sixth aspect, wherein a display portion is further included. The display portion can display the processing status.

This deodorization functional agent reproducing process further includes a display portion that can display the processing status of the reproducing process detected by the processing status detection means. In addition, the "display portion" referred here is a portion that is formed by a liquid crystal display panel, LED, some other display device, or a combination of these.

Therefore, the processing status of a reproducing process can be checked visually. Consequently, a user can check the processing status in real time, and a reproducing process can be performed according to the status.

The deodorization function reproducing device in accordance with an eight aspect of the present invention is the deodorization function reproducing device of any of the fourth to the seventh aspects, wherein a switch is further included. The switch is able to judge the start and end of a reproducing process. The active species generating portion starts generating active species when the switch becomes ON and stops generating active species when the switch becomes OFF.

This deodorization functional agent reproducing process further includes a switch that is able to judge the start and end of a reproducing process of a deodorization functional agent. The active species generating portion starts generating active species when the switch becomes ON and stops generating active species when the switch becomes OFF.

Therefore, with this deodorization functional agent reproducing process, a reproducing process of a deodorization functional agent can be started or ended by turning the switch ON or OFF. Consequently, a user can decide the timing for starting and ending a reproducing process of the deodorization functional agent based on his or her own judgment.

The deodorization function reproducing device in accordance with a ninth aspect of the present invention is the deodorization function reproducing device of any of the fourth to the eighth aspects, wherein an adjustment switch is further included. The adjustment switch can adjust the amount that the active species are generated. The active species generating portion increases the amount that the active species are generated when the adjustment switch becomes ON and decreases the amount that the active species are generated when the adjustment switch becomes OFF.

With this deodorization function reproducing device, the adjustment switch is able to adjust the increasing and the decreasing of the amount that the active species are generated by the active species generating portion. The active species generating portion increases the amount that the active species are generated when the adjustment switch becomes ON and decreases the amount that the active species are generated when the adjustment switch becomes OFF.

Therefore, when using active species other than for the deodorization functional agent and the like, the amount that the active species are generated can be increased or decreased to only an amount for the reproducing process of the deodorization functional agent, by turning the adjustment switch ON or OFF. As a result, even if the adjustment switch is OFF, the generation of active species can be suppressed without being terminated. For this reason, it is possible to prevent energy from being used unnecessarily.

The deodorization function reproducing device in accordance with a tenth aspect of the present invention is the deodorization function reproducing device of the eight aspect, wherein the switch or the adjustment switch can be turned ON and OFF by the opening and closing of a door from which the deodorization functional agent included in the casing can be put in or taken out.

With this deodorization function reproducing device, the door included in the casing acts as the switch or the adjustment switch. Therefore, by opening or closing the door, the switch or the adjustment switch can be turned ON or OFF. For this reason, the active species generating portion can judge whether to start or end a reproducing process of the deodorization functional agent through the door being opened or closed. In addition, the active species generating portion can increase or decrease the amount that the active species are generated through the door being opened or closed. As a result, it is not necessary to create a component that forms the switch or the adjustment switch, and manufacturing costs can be suppressed. Furthermore, since the switch or the adjustment switch becomes OFF when the door is opened, when a user opens the door, the generation of active species can be stopped or suppressed by the active species generating portion. For this reason, a user can operate safely.

The deodorization function reproducing device in accordance with an eleventh aspect of the present invention is the deodorization function reproducing device of any of the eighth to the tenth aspects, wherein the switch or the adjustment switch can be turned ON or OFF by putting in or taking out the deodorization functional agent, in or out of the casing.

With this deodorization function reproducing device, the switch or the adjustment switch can be turned ON or OFF through the deodorization functional agent being put in or taken out of the casing. Therefore, if the deodorization functional agent is not inside the casing, it is possible to prevent electricity from being discharged unnecessarily. For this reason, energy can be prevented from being used unnecessarily.

The deodorization function reproducing device in accordance with a twelfth aspect of the present invention is the deodorization function reproducing device of any of the first to the eleventh aspects, wherein a deodorization functional agent is further included, and the deodorization function reproducing device forms an air conditioner. The deodorization functional agent adsorbs at least one of either the odor molecules or harmful gas components floating in the air.

This deodorization function reproducing device forms an air conditioner, and further includes a deodorization functional agent for adsorbing at least one of either the odor molecules or harmful gas components floating in the air. Therefore, this deodorization function reproducing device can deodorize by adsorbing at least one of either the odor molecules or harmful gas components floating in the air. Furthermore, since the deodorization functional agent, which needed to be replaced in every predetermined period of time in the past, can be reused, it is possible to suppress generation of waste, generation of polluted water from washing the deodorization functional agent with water, and the like. For this reason, when a reproducing process of the deodorization functional agent is performed, burden on the environment can be reduced. In addition, usability can be improved, since reproduction can be done reliably in a short period of time and with a simple operation.

The air conditioner in accordance with a thirteenth aspect of the present invention includes a deodorization function reproducing device. The deodorization function reproducing device includes a casing and an active species generating portion. The casing can store a deodorization functional agent. The active species generating portion generates active species to decompose or inactivate at least one of either the odor molecules or harmful gas components.

This air conditioner includes a deodorization function reproducing device that is able to reproduce a deodorization functional agent that has been used, through active species generated by the active species generating portion.

Therefore, this air conditioner can decompose odor molecules and harmful gas components adsorbed by the deodorization functional agent, if the deodorization functional agent is inside the casing. Consequently, this can also contribute to an improvement in the deodorization performance of this air conditioner.

### EFFECT OF THE INVENTION

With the deodorization function reproducing device of the first aspect, it is possible to suppress the generation of waste, generation of polluted water from washing a deodorization functional agent with water, and the like, because the deodorization functional agent, which needed to be replaced in every predetermined period of time in the past, can be reused. For this reason, when a reproducing process of the deodorization functional agent is performed, burden on the environment can be reduced. In addition, since reproduction can be done reliably in a short period of time and with a simple operation, usability can be improved.

With the deodorization function reproducing device of the second aspect, it is possible to accelerate the exposure of active species to the deodorization functional agent. Consequently, the effect of the reproducing process of the deodorization functional agent can be improved.

With the deodorization function reproducing device of the third aspect, it is possible to perform the reproducing process of the deodorization functional agent using purified air.

With the deodorization function reproducing device of the fourth aspect, the reproducing process of the deodorization functional agent ends when a predetermined period of time has passed. Consequently, after a reproducing process of the deodorization functional agent is started, the reproducing process of the deodorization functional agent can be ended without the user having to perform an operation for terminating it.

With the deodorization function reproducing device of the fifth aspect, a user can adjust the reproducing process level according to the degree of uncleanness of the deodorization functional agent by setting a reproducing process time. Consequently, since it is possible to suppress a reproducing process from being performed excessively for the degree of uncleanness of the deodorization functional agent, it is possible to suppress energy used for the reproducing process. In addition, a reproducing process that is insufficient for the degree of uncleanness of the deodorization functional agent can be resolved, and thereby the deodorization functional agent can be reproduced more reliably.

With the deodorization function reproducing device of the sixth aspect, the active species generating portion can adjust the reproducing time according to the processing status detected by the processing status detection means. Consequently, a reproducing process that is suitable for the degree of uncleanness of the deodorization functional agent can be performed automatically. As a result, it is not necessary for a user to set a reproducing process time, and operational burden on the user can be reduced, and a reproducing process can be performed easily. In addition, since it is possible to suppress a reproducing process from being performed excessively for the degree of uncleanness of the deodorization functional agent, it is possible to suppress energy used for the reproducing process. Moreover, since an insufficiency of the reproducing process for the degree of uncleanness of the deodorization functional agent can be resolved, the deodorization functional agent can be reproduced more reliably.

With the deodorization function reproducing device of the seventh aspect, the processing status of a reproducing process can be checked visually. For this reason, a user can check the processing status in real time, and a reproducing process can be performed according to the status.

With the deodorization function reproducing device of the eighth aspect, in the deodorization functional agent reproducing process, a reproducing process of the deodorization functional agent can be started or ended by turning the switch ON or OFF. Consequently, a user can decide the timing for starting and ending the reproducing process of the deodorization functional agent based on his or her own judgment.

With the deodorization function reproducing device of the ninth aspect, when using active species other than for the deodorization functional agent and the like, the amount that the active species are generated can be increased or decreased to only an amount for the reproducing process of the deodorization functional agent, by turning the adjustment switch ON or OFF. As a result, even if the adjustment switch is OFF, the generation of active species can be suppressed without being terminated. For this reason, it is possible to prevent energy from being used unnecessarily.

With the deodorization function reproducing device of the tenth aspect, by opening or closing the door, the switch or the adjustment switch can be turned ON or OFF. For this reason, the active species generating portion can judge whether to start or end a reproducing process of the deodorization functional agent through the door being opened or closed. In addition, the active species generating portion can increase or decrease the amount that the active species are generated through the door being opened or closed. As a result, it is not necessary to create a component that forms the switch or the adjustment switch, and manufacturing costs can be suppressed. Furthermore, since the switch or the adjustment switch becomes OFF when the door is opened, when a user opens the door, the generation of active species can be stopped or suppressed by the active species generating portion. For this reason, a user can operate safely.

With the deodorization function reproducing device of the eleventh aspect, if the deodorization functional agent is not inside the casing, it is possible to prevent electricity from being discharged unnecessarily. For this reason, energy can be prevented from being used unnecessarily.

With the deodorization function reproducing device of the twelfth aspect, the deodorization function reproducing device is able to deodorize by adsorbing at least one of either the odor molecules or harmful gas components floating in the air. Furthermore, since the deodorization functional agent, which needed to be replaced in every predetermined period of time in the past, can be reused, it is possible to suppress generation of waste, generation of polluted water from washing the deodorization functional agent with water, and the like. For this reason, when a reproducing process of the deodorization functional agent is performed, burden on the environment can be reduced. In addition, usability can be improved, since reproduction can be done reliably in a short period of time and with a simple operation.

With the air conditioner of the thirteenth aspect, odor molecules and harmful gas components adsorbed by the deodorization functional agent can be decomposed if the deodorization functional agent is inside the casing. Consequently, this can also contribute to an improvement in the deodorization performance of this air conditioner.

### BRIEF DESCRIPTION OF THE DRAWINGS

Fig. 1 is a diagram showing a deodorization function reproducing device in accordance with a first embodiment;
Fig. 2(a) is a perspective view showing the structure on the upstream side in the air flow direction of a streamer discharger;
Fig. 2(b) is a perspective view showing the shape of a streamer discharging electrode;
Fig. 2(c) is a top layout drawing of the streamer discharger;
Fig. 2(d) is a diagram showing the appearance of a streamer discharge;
Fig. 3 is a schematic block diagram of a control unit in accordance with the first embodiment;
Fig. 4 is an appearance perspective view of an air purifier in accordance with a second embodiment;
Fig. 5 is an exploded perspective view of a deodorizing agent casing, types of filters, and a blower in accordance with the second embodiment;
Fig. 6(a) is a perspective view showing the structure on the upstream side in the air flow direction of a streamer discharger in accordance with the second embodiment;
Fig. 6(b) is a top layout drawing of the streamer discharger in accordance with the second embodiment;
Fig. 7 is a schematic block diagram of a control unit in accordance with the second embodiment;
Fig. 8 is a diagram showing a schematic configuration of a refrigerant circuit of an air conditioner in accordance with a third embodiment and the flow of air;
Fig. 9 is a side section of an indoor device;
Fig. 10 is a schematic block diagram of a control unit in accordance with the third embodiment;
Fig. 11 is an appearance perspective view of an air purifier in accordance with a fourth embodiment;
Fig. 12 is an appearance perspective view of the air purifier in a state that a front panel is removed;
Fig. 13 is an appearance perspective view of a rear side of the air purifier;
Fig. 14 is a diagram showing the position of a fan of the air purifier;
Fig. 15 is a diagram for describing air purification through a main airflow and a bypass airflow;
Fig. 16(a) is a front view showing the main airflow path and the bypass airflow path;
Fig. 16(b) is a side view showing the main airflow path and the bypass airflow path;
Fig. 16(c) is a top view showing the main airflow path and the bypass airflow path;
Fig. 17 is a configuration diagram of a unit storage portion;
Fig. 18 is an appearance perspective view of a streamer discharging unit;
Fig. 19 is a top section view of the streamer discharging unit;
Fig. 20 is a rear perspective view of a plasma ion-wire;
Fig. 21 is a top view of the plasma ion-wire;
Fig. 22 is a front view showing when a prefilter is mounted on the plasma ion-wire;
Fig. 23 is an enlarged view of a main portion of the prefilter;
Fig. 24 is an enlarged view of a main portion of the plasma ion-wire;
Fig. 25 is a schematic block diagram of a control unit in accordance with the fourth embodiment;
Fig. 26 is a diagram showing a blower in accordance with a modified example (D); and
Fig. 27 is a diagram showing an outward extending pathway in accordance with a modified example (H).

### DESCRIPTION OF THE REFERENCE SYMBOLS

- 1, 1a, 1b: Deodorization function reproducing device
- 2, 20, 67: Deodorizing agent casing (casing)
- 2b, 20b: Door
- 3, 3a, 3b: Streamer Discharger (active species generating portion)
- 4, 4a, 4b: Deodorizing agent (deodorization functional agent)
- 5,44: Blower
- 6, 50, 90: Control unit
- 6c to 6e, 50c to 50f, 92c to 92e: Various sensors (processing status detection means)
- 7,51: Switch
- 52: Adjustment switch
- 8, 53: Display panel (display portion)
- 100: Air conditioner
- 200: Air purifier
- 212: Blow out opening
- 213: Lower suction opening
- 214: Side suction openings
- 231: Blower fan
- 240: Air purifying unit (first purifying processing portion)
- 241: Prefilter
- 241 a: Filter portion (first pass through portion)
- 241b: Divider ventilation portion (filter divider portion)
- 241O: Holes (second pass through portions)
- 242: Plasma ion-wire (downstream member)
- 243: photocatalytic filter
- 244: Plasma catalytic filter
- 263: Streamer discharging unit

### BEST MODE FOR CARRYING OUT THE INVENTION

### <First Embodiment>

### <Configuration of a Deodorization Function Reproducing Device>

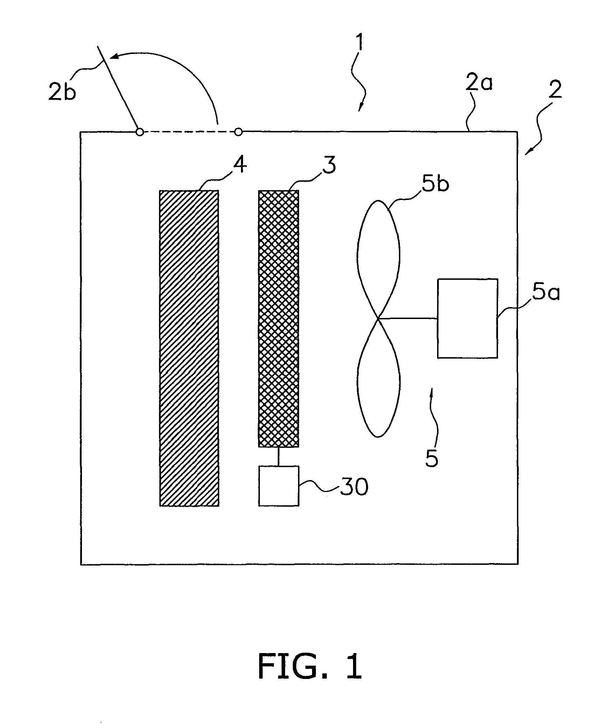

The appearance of a deodorization function reproducing device 1 in accordance with a first embodiment of the present invention is shown in Fig. 1. The deodorization function reproducing device 1 performs a reproducing process of a deodorizing agent 4, which the deodorizing effect thereof lowers after being used for a certain period of time. This deodorization function reproducing device 1 includes a deodorizing agent casing 2, a streamer discharger 3, a high voltage unit 30, a blower 5, and a control unit 6.

### (1) Deodorizing Agent Casing

The deodorizing agent casing 2 forms the outside surface of the deodorization function reproducing device 1, and includes the steamer discharger 3, the blower 5, and the control unit 6 inside thereof. The deodorizing agent casing 2 is made of a main body portion 2a and a door 2b. The door 2b can be opened and closed, and the deodorizing agent 4 can be taken out or put in from the opening of the deodorizing agent casing 2 when the door 2b is opened.

### (2) Steamer Discharger

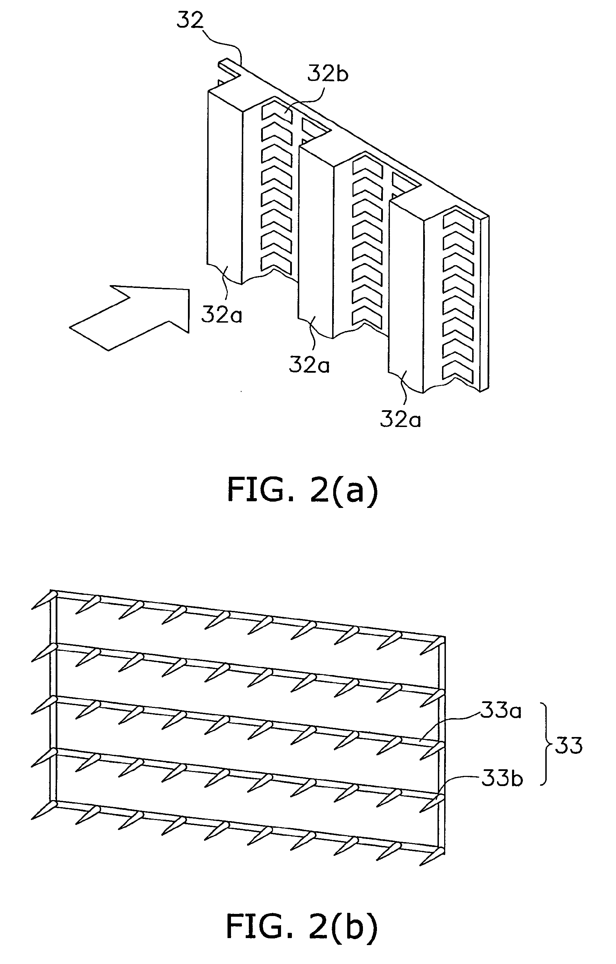

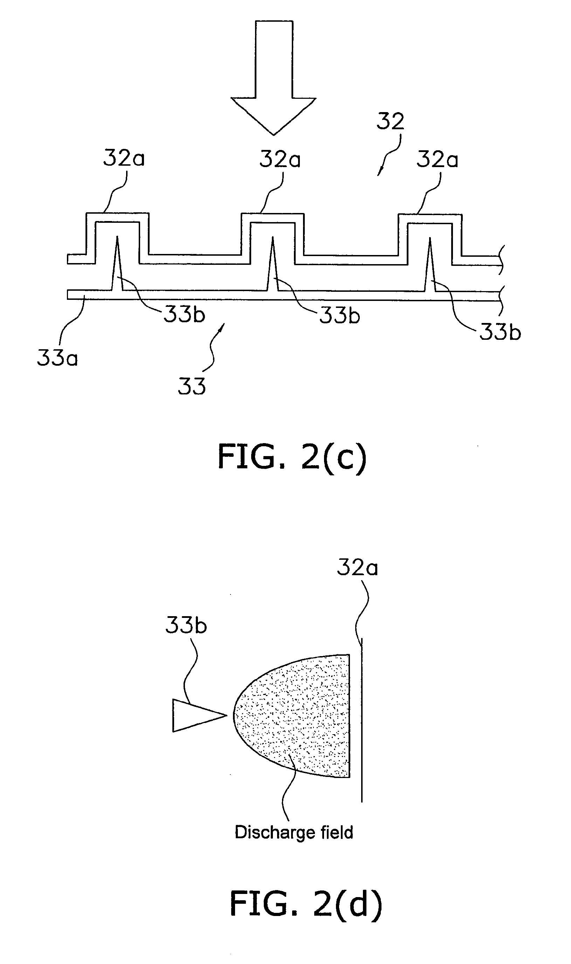

As shown in Figs. 2(a), 2(b), and 2(c), the steamer discharger 3 is mainly made up of an opposite electrode 32, and a streamer discharging electrode 33. The opposite electrode 32 is a metal plate having a section in a square corrugated shape, and is composed of actual electrode portions 32a that functions substantially as electrodes, and a plurality of slit portions 32b. In addition, the slit portions 32b are provided so that air flows to the rear side (refer to the outlined arrow in Fig. 2(a)). The streamer discharging electrode 33 is composed of electrode bars 33a and needle electrodes 33b. The needle electrodes 33b are fixed to be roughly orthogonal to the electrode bars 33a. As shown in Fig. 2(c), this streamer discharging electrode 33 is disposed on a downstream side of the opposite electrode 32 in the air flow direction (refer to the outlined arrow in Fig. 2(c)). Furthermore, at this time, the needle electrodes 33b of the streamer discharging electrode 33 are disposed to be facing to the actual electrode portions 32a of the opposite electrode 32.

In this streamer discharger 3, a streamer discharge shown in Fig. 2(d) occurs between the streamer discharging electrode 33 and the opposite electrode 32, when a direct current, alternate current or pulsed discharge voltage is applied between the streamer discharging electrode 33 and the opposite electrode 32. By doing so, cool plasma is generated in the discharge field when the streamer discharge occurs. Moreover, due to this cool plasma, radical species such as high speed electrons, ions, ozone, and hydroxy radical, and other excited molecules (excited oxygen molecules, excited nitrogen molecules, excited water molecules), and the like are generated. Furthermore, these active species flow with the air and are supplied to the deodorizing agent 4, and the performance of deodorization of the deodorizing agent 4 is reproduced.

In addition, these active species have extremely high energy level, and have the ability to deodorize and decompose small organic molecules, such as ammonias, aldehydes, nitrogen oxide, that are included in the air.

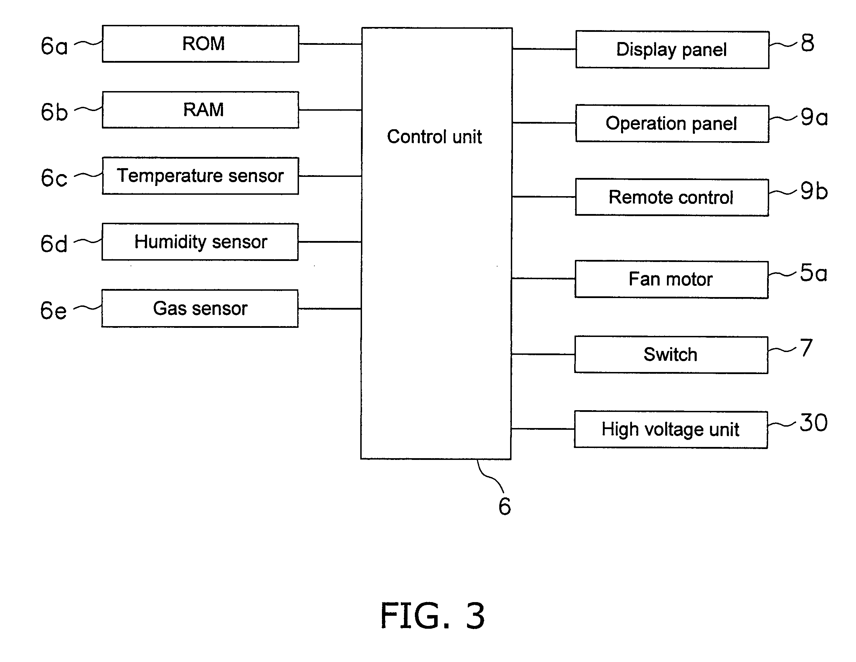

Moreover, the high voltage unit 30 is connected to the steamer discharger 3. The high voltage unit 30 can adjust the amount that the active species are generated by the streamer discharger 3, by controlling the amount of discharge thereof (refer to Figs. 1 and 3).

### (3) Blower

The blower 5 sends the active species generated by the streamer discharger 3 to the deodorizing agent 4. As shown in Fig. 1, this blower 5 includes a fan motor 5a and a blower fan 5b. This blower fan 5b is driven to rotate by the fan motor 5a. An inverter motor, which frequency is controlled by an inverter circuit, is adopted as the fan motor 5a.

### (4) Control Unit

The deodorization function reproducing device 1 further includes the control unit 6 made up of a microprocessor. As shown in Fig. 3, a ROM 6a in which a control program and various parameters are stored, and a RAM 6b in which variables and the like in process are temporarily stored, and the like are connected to the control unit 6. In addition, a temperature sensor 6c, a humidity sensor 6d, a gas sensor 6e, a switch 7, a display panel 8, an operation panel 9a, and a remote control 9b are connected to the control unit 6.

The detected signals of each of the sensors, of the various types of the sensors of the temperature sensor 6c, humidity sensor 6d, and the gas sensor 6e, are outputted to the control unit 6. The gas sensor 6e can measure the gas concentration generated from harmful gas components and the like, and odor molecules decomposed by the active species. Through these various types of sensors, the reproducing status of the deodorizing agent 4 is evaluated and the reproducing time can be automatically adjusted.

The switch 7 turns the discharge of the streamer discharger 3 ON and OFF. The switch 7 is OFF when the door 2b is opened, and ON when the door 2b is closed. Thus, the reproducing process of the deodorizing agent 4 can be started or terminated by opening or closing the door 2b. Furthermore, when a user opens the door 2b, the generation of active species by the streamer discharger 3 is stopped because the switch is OFF when the door 2b is opened. For this reason, a user can operate safely.

The display panel 8 displays monitor information (reproducing process information) through the various sensors 6c to 6e, timer information, maintenance information, and the like, and can be seen by a user and the like from the outside through a display panel opening (not shown). In addition, this display panel 8 can be made up of a liquid crystal display panel, LED, some other display device, or a combination of these.

A user can turn the switch 7 ON or OFF through the operation panel 9a and the remote control 9b. Furthermore, a user can change the operation mode of deodorizing agent reproducing operation and deodorizing operation and the like, adjust the processing time of reproducing operation, start or terminate an operation in the various operation modes, and so forth, through the operation panel 9a and the remote control 9b.

Furthermore, the control unit 6 is connected to the fan motor 5a and the high voltage unit 30. The control unit 6 can control the air volume through the fan motor 5a and the discharge amount related to the generation of active species through the high voltage unit 30, according to the detected results and the like of the various sensors 6c to 6e and the operation of the user.

### <Characteristics of the First Embodiment>

(1)
   In this deodorization function reproducing device 1, the deodorizing agent 4 used in any preferred location is installed in the interior of the deodorizing agent casing 2. In addition, this device generates active species through the streamer discharger 3. At least one of either the odor molecules adsorbed by the deodorizing agent 4 or the harmful gas components adsorbed by the deodorizing agent 4 is decomposed by the active species, and the deodorizing agent 4 is reproduced.
   Consequently, since the deodorizing agent 4, which needs to be changed in every predetermined period of time in the past, can be reused, it is possible to suppress the generation of waste, polluted water from washing it, and the like, and thereby can be low environment impact when the reproducing process of the deodorizing agent 4 is performed. In addition, usability can be improved because reproducing can be done reliably in a short period of time with a simple operation.
(2)
   This deodorization function reproducing device 1 further includes the blower 5 for generating airflow inside the deodorizing agent casing 2. Consequently, the deodorizing agent 4 can be exposed effectively to active species, and thereby the effect of the reproducing process of the deodorizing agent 4 can be improved.
(3)
   This deodorization function reproducing device 1 generates active species for a predetermined time in the streamer discharger 3. Consequently, the reproducing process of the deodorizing agent 4 ends after a predetermined time is passed. For this reason, a user can terminate the reproducing process of the deodorizing agent 4 without an operation for ending, after the reproducing process of the deodorizing agent 4 is started.
   In addition, the predetermined time for generating the active species can be adjusted. The reproducing level of the deodorizing agent 4 is higher if the reproducing process time is longer, and is lower if the reproducing process time is shorter. Consequently, a user can adjust the reproducing process level according to the degree that the deodorizing agent 4 is contaminated, by setting the time of the reproducing process. Thus, energy used for the reproducing process can be held down because overly performing the reproducing process, for the degree that the deodorizing agent 4 is contaminated, can be prevented. In addition, since an insufficient reproducing process, for the degree that the deodorizing agent 4 is contaminated, can be prevented, the deodorizing agent 4 can be reproduced more reliably.
(4)
   This deodorization function reproducing device 1 further includes the temperature sensor 6c, the humidity sensor 6d, the gas sensor 6e, and the like, that are capable of detecting the processing status of the reproducing process. Consequently, the streamer discharger 3 can adjust the predetermined time according to the processing status detected by these various sensors 6c to 6e. For this reason, a reproducing process suitable for the degree that the deodorizing agent 4 is contaminated can be automatically performed. By doing so, a user does not have to set a reproducing process time, and thereby a user can operate with less load, and the operation of the reproducing process can be performed easily. Furthermore, energy used for the reproducing process can be held down because overly performing the reproducing process, for the contamination of the deodorizing agent 4, can be prevented. In addition, since an insufficiency of the reproducing process, for the contamination of the deodorizing agent 4, can be enough, the deodorizing agent 4 can be reproduced more reliably.
   In addition, this deodorization function reproducing device 1 further includes the display panel 8 that is capable of displaying the processing status of the reproducing process detected by the various sensors 6c to 6e. Consequently, the processing status of the reproducing process can be confirmed visually. Thus, a user can confirm the processing status in real time, and can perform the reproducing process according to the status.
(5)
   This deodorization function reproducing device 1 further includes the switch 7 able to be switching the start and the end of a reproducing process of the deodorizing agent 4. The streamer discharger 3 starts the generation of active species when the switch 7 becomes ON, and ends the generation of active species when the switch 7 becomes OFF. Consequently, in this deodorizing agent reproducing process, the start and the end of a reproducing process of the deodorizing agent 4 can be performed by the ON and OFF of the switch 7. Accordingly, a user can decide the timing to start and end the reproducing process based on his or her own judgment.
   In addition, in this deodorization function reproducing device 1, the door 2b included in the deodorizing agent casing 2 plays the role of the switch 7. Consequently, the switch 7 can be turned ON or OFF by opening or closing the door 2b. Thus, the streamer discharger 3 can judge the start and the end of a reproducing process of the deodorizing agent 4 through the opening and closing of the door 2b. As a result, it is not necessary to create a component to form the switch 7, and thereby manufacturing costs can be kept down. Furthermore, since the switch 7 is OFF when the door 2b is opened, the generation of active species by the streamer discharger 3 is stopped when the user opens the door 2b. For this reason, a user can operate safely.

### <Second Embodiment>

### <Overall Configuration of an Air Purifier>

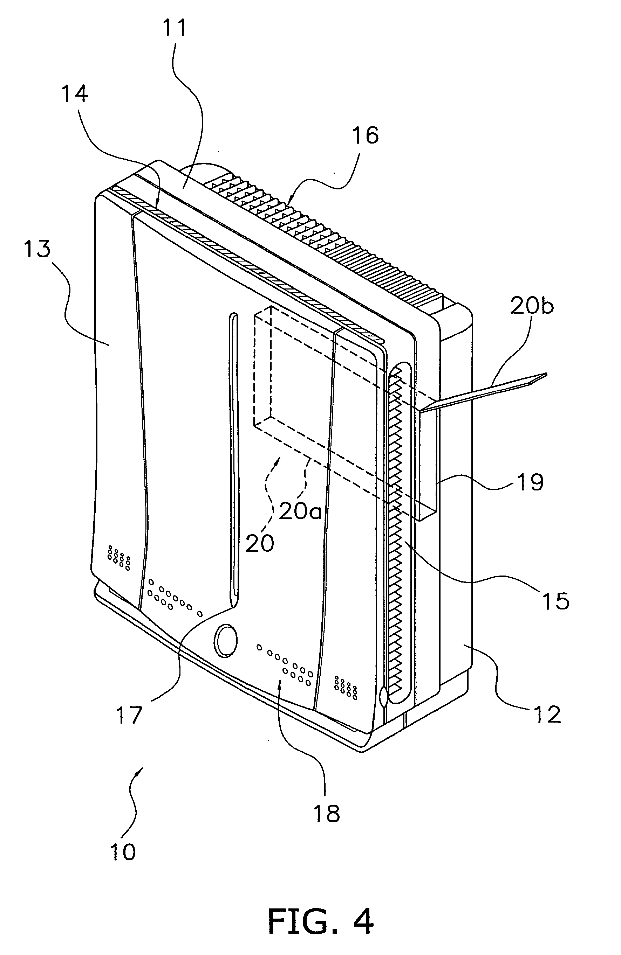

An appearance diagram of an air purifier 10 in accordance with a second embodiment is shown in Fig. 4.

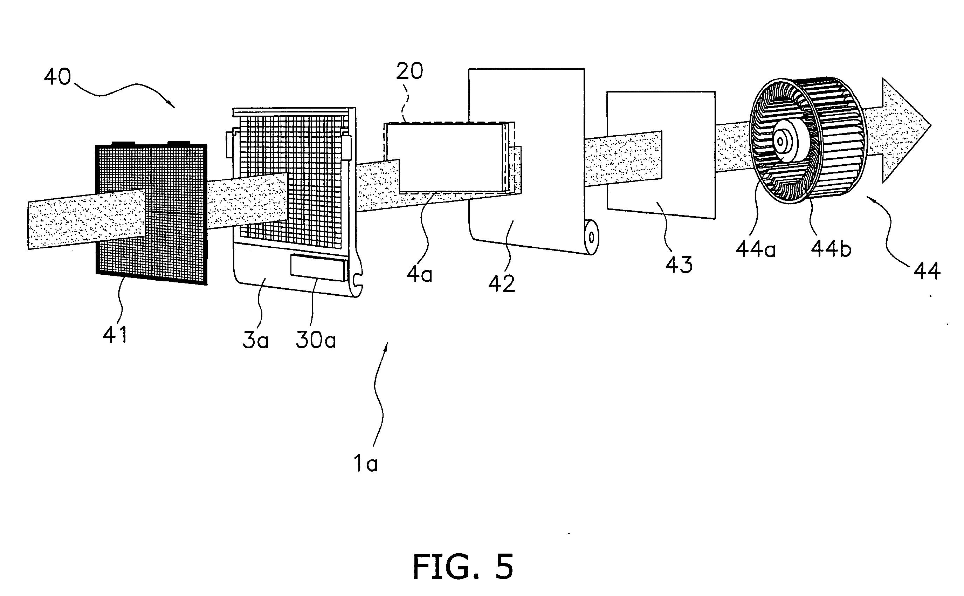

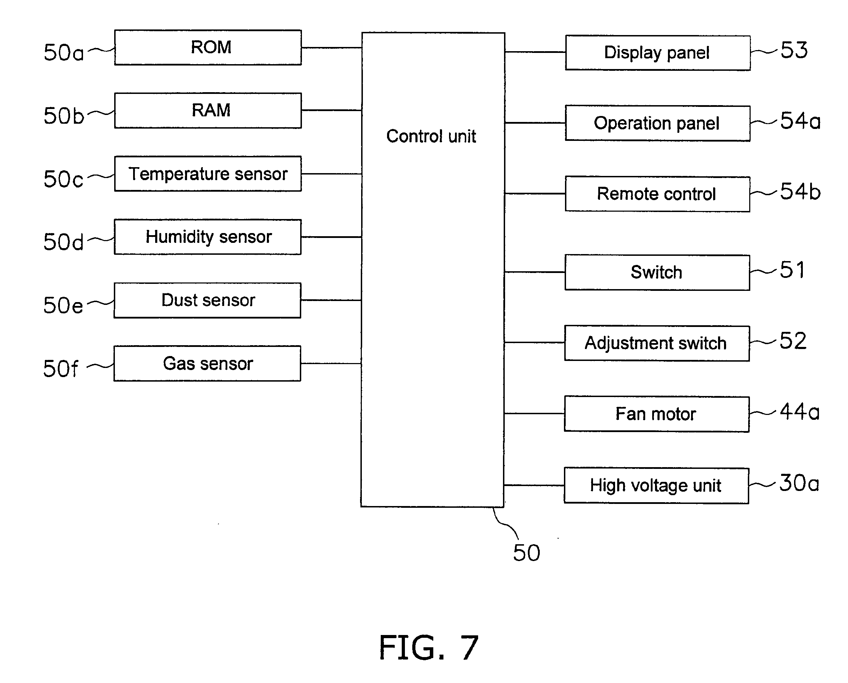

The air purifier 10 keeps an indoor environment comfortable by purifying the indoor air and sending purified air into the interior of an office building, a residence, and the like. This air purifier 10 includes an air purifier casing 11, a blower 44 (refer to Fig. 5), a control unit 50 (refer to Fig. 7), and a filter unit 40 (refer to Fig. 5).

### <Components of the Air Purifier>

### (1) Air Purifier Casing

The air purifier casing 11 forms the outside surface of the air purifier 10, and includes the blower 44, the control unit 50, and the filter unit 40 inside thereof. The air purifier casing 11 has a main body portion 12 and a front panel 13.

### A. Main Body Portion

The main body portion 12 includes a top suction opening 14, side suction openings 15, and a supply opening 16. The top suction opening 14 and the side suction openings 15 are roughly rectangular openings for drawing in the interior air into the air purifier 10, in order to purify the indoor air inside the air purifier 10. The top suction opening 14 is provided on the front side edge portion on top of the main body portion 12, same as the surface on which the supply opening 16 is provided. The side suction openings 15 are a pair of openings provided on the left and right side surfaces of the main body portion 12. The supply opening 16 is provided on the rear side edge portion on top of the main body portion 12. The supply opening 16 is an opening for blowing out purified air into the interior space from the air purifier 10. In addition, the main body portion 12 has an opening portion 19 on the side surface thereof, from which a deodorizing agent 4a can be taken out or put in. A deodorizing agent casing 20 to be described later is stored in the opening portion.

### B. Front Panel

The front panel 13 is provided on the front of the main body portion 12, and covers the filter unit 40 installed in the interior of the main body portion 12. The front panel 13 has a front suction opening 17 and a display panel opening 18. The front suction opening 17 is a roughly rectangular opening provided approximately on the center portion of the front panel 13 for drawing in interior air inside the air purifier 10. The display panel opening 18 is provided so that a display panel 53 to be described later can be seen from outside the air purifier casing 11.

### (2) Blower

The blower 44 draws in the indoor air from each of the suction openings (top suction opening 14, side suction openings 15, and front suction opening 17), and blows out purified air from the supply opening 16. This blower 44 is provided inside the air purifier casing 11, and is configured so that indoor air drawn in from each of the suction openings passes through the filter unit 40. In addition, as shown in Fig. 5, the blower 44 includes a fan motor 44a and a blower fan 44b. This blower fan 44b is driven to rotate by the fan motor 44a. An inverter motor, which the frequency thereof is controlled by an inverter circuit, is adopted as the fan motor 44a. A centrifugal fan is adopted as the blower fan 44b.

### (3) Control Unit

The air purifier 10 further includes the control unit 50 made up of a microprocessor. As shown in Fig. 7, a ROM 50a in which a control program and various parameters are stored, and a RAM 50b in which variables and the like in process are temporarily stored, and the like are connected to the control unit 50. In addition, a temperature sensor 50c, a humidity sensor 50d, a dust sensor 50e, a gas sensor 50f, a switch 51, an adjustment switch 52, and a display panel 53 are connected to the control unit 50.

The detected signals of each of the sensors, of the various types of the sensors of the temperature sensor 50c, humidity sensor 50d, the dust sensor 50e, and the gas sensor 50f are outputted to the control unit 50. The dust sensor 50e can measure the particle concentration of dust and the like by irradiating the air that is brought in with light, and detect the amount of light that is reflected diffusely by smoke, dusts, pollen, or other particles included in the air, and reached the light receiving element. The gas sensor 50f can measure the gas concentration generated from harmful gas components and the like decomposed by the active spieces, and odor molecules decomposed by the active species. Through these various types of sensors, the reproducing status of the deodorizing agent 4a is evaluated and the reproducing time can be automatically adjusted.

With the switch 51, a reproducing process of the deodorizing agent 4a can be started or ended. The switch 51 is OFF when the door 20b is opened, and the switch 51 is ON when the door 20b is closed. Thus, the generation of active species by the streamer discharger 3a is stopped when a user opens the door 20b. For this reason, a user can operate safely.

In addition, the adjustment switch 52 is operated for increasing or decreasing the amount of active species generated by the streamer discharger 3a. The adjustment switch 52 is OFF when the deodorizing agent a is taken out from the interior of the deodorizing agent casing 20, and is ON when the deodorizing agent 4a is in the deodorizing agent casing 20. The amount of active species generated in the streamer discharger 3a decreases when the adjustment switch 52 is OFF, and amount of active species generated in the streamer discharger 3a increases when the adjustment switch 52 is ON. Accordingly, if the deodorizing agent 4a is not inside the deodorizing agent casing 20, only the amount of active species for the various filters can be provided. For this reason, the amount of active species generated for the reproducing process of the deodorizing agent 4a can be increased or decreased by turning the adjustment switch 52 ON or OFF.

The display panel 53 displays the operation mode, monitor information through the various sensors 50c to 50f, timer information, maintenance information, and the like, and can be seen by a user and the like from the outside through a display panel opening 18. In addition, this display panel 53 can be made up of a liquid crystal display panel, LED, some other display device, or a combination of these.

A user can turn the switch 51 and the adjustment switch 52 ON and OFF through the operation panel 54a and the remote control 54b. Furthermore, a user can change the operation mode of deodorizing agent reproducing operation and deodorizing operation and the like, adjust the processing time of reproducing operation, start or terminate an operation in the various operation modes, and so forth, through the operation panel 54a and the remote control 54b.

Furthermore, the control unit 50 is connected to the fan motor 44a and the high voltage unit 30a. The control unit 50 can control the air volume through the fan motor 44a and the discharge amount related to the generation of active species through the high voltage unit 30a, according to the detected results of the various sensors 50c to 50f and the operation of the user and the like.

### (4) Filter Unit

The filter unit 40 is provided in the interior of the air purifier casing 11, and removes fine particles in the indoor air that is drawn in from each of the suction openings 14, 15, and 17. As shown in Fig. 5, the filter unit 40 includes a prefilter 41, the streamer discharger 3a, the deodorizing agent casing 20, a photocatalytic filter 42, and a plasma catalytic filter 43. The filter unit 40 is configured so that the indoor air drawn in from each of the suction openings 14, 15, and 17 passes through the prefilter 41, the streamer discharger 3a, the deodorizing agent casing 20, the photocatalytic filter 42, and the plasma catalytic filter 43 in this order, inside the filter unit 40.

### A. Prefilter

The prefilter 41 is a filter for removing comparatively large dusts and the like from the air drawn inside the air purifier casing 11 by the blower 44. The prefilter 41 includes a net portion and a frame (not shown). The net portion is a threadlike resin net made of polypropylene (hereinafter referred to as PP), and on which comparatively large dusts and the like in the air drawn inside the air purifier casing 11 are attached. Furthermore, the fabric that makes up the net portion is composed of a covering layer made by PP, same as a core made by PP. A catechin and photocatalyst of visible light type are supported on the covering layer to be exposed on the air side. The photocatalyst of visible light type includes oxidized titanium and the like that a photocatalytic effect is activated by visible light, and removes bacteria and viruses of mold bacteria, bacteria, and the like in dusts and the like that are attached to the net portion. Catechin is a type of polyphenol, and is the generic name of epicatechin, epigallocatechin, epicatechin gallate, epigallocatechin gallate, and the like. This catechin suppresses the multiplication of bacteria of mold bacteria, bacteria, and the like in the dusts and the like attached on the net portion, and inactivates viruses.

### B. Streamer Discharger

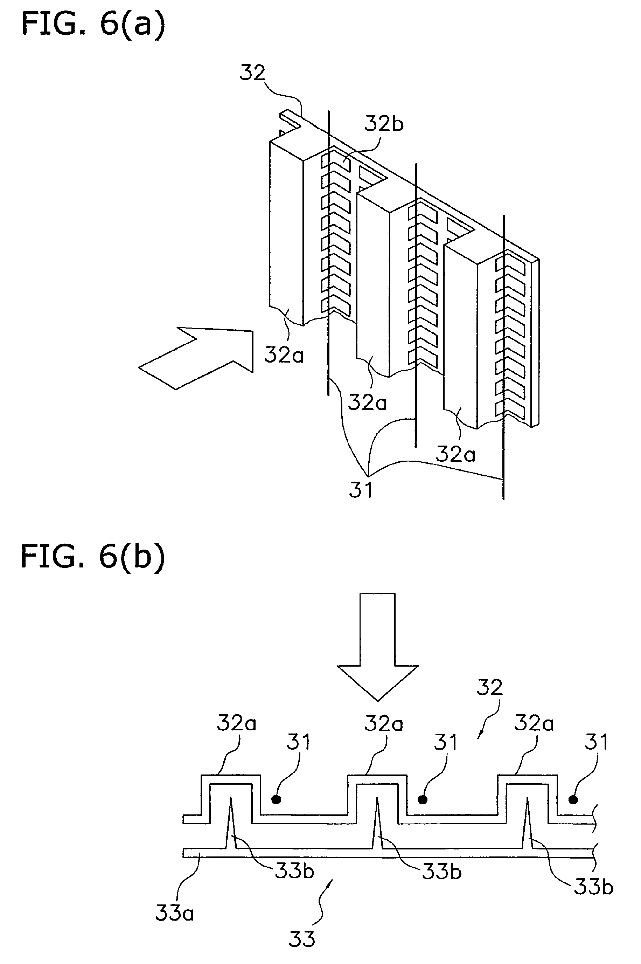

As shown in Figs. 2(b), 6(a), and 6(b), the streamer discharger 3a is mainly made up of an opposite electrode 32, ion-wires 31, and a streamer discharging electrode 33. The opposite electrode 32 is a metal plate having a section in a square corrugated shape, and is composed of actual electrode portions 32a that function substantially as electrodes, and a plurality of slit portions 32b. In addition, the slit portions 32b are provided so that air flows to the rear side (refer to the outlined arrow in Fig. 6(a)). The ion-wires 31 are disposed on an upstream side of the opposite electrode 32 in the air flow direction (refer to the outlined arrow in Fig. 6(a)). In addition, at this time, the ion-wires 31 are each disposed between the actual electrode portions 32a. Moreover, the ion-wire 31 is formed by a tungsten wire rod and the like of extremely small diameter, and is used as a discharging electrode. The streamer discharging electrode 33 is composed of electrode bars 33a and needle electrodes 33b. The needle electrodes 33b are fixed to be roughly orthogonal to the electrode bars 33a. As shown in Fig. 6(b), this streamer discharging electrode 33 is disposed on a downstream side of the opposite electrode 32 in the air flow direction (refer to the outlined arrow in Fig. 6(b)). Furthermore, at this time, the needle electrodes 33b of the streamer discharging electrode 33 are disposed to be opposite from the actual electrode portions 32a of the opposite electrode 32.

In addition, of the electrodes 31, 32, and 33, the opposite electrode 32 and the ion-wire 31 are provided for charging comparatively small dusts that are floating in the air, which have passed the prefilter 41. On the other hand, the opposite electrode 32 and the streamer discharging electrode 33 are provided for generating active species to be provided to a titanium apatite support filter 31 to be described later. Hereinafter, each of the combinations of the electrodes will be described in detail.

### (Opposite Electrode and Ion-wire)

In this streamer discharger 3a, when a high voltage is applied between the ion-wires 31 and the actual electrode portions 32a, discharge occurs between the two electrodes 31 and 32. As a result, dusts and the like that pass through between the two electrodes 31 and 32 are charged with plus electrical charge. The charged dusts are supplied to the back via the slit portions 32b, and are electrostatically adsorbed by an electrostatic filter (not shown), to be described later. Furthermore, at this time, since viruses, bacteria, and the like included in the dusts are also charged, the absorption efficiency of viruses and bacteria to a titanium apatite (not shown) to be described later improves.

### (Opposite Electrode and Streamer Discharging Electrode)

In this streamer discharger 3a, when a direct current, alternate current, or a pulsed discharge voltage is applied between the streamer discharging electrode 33 and the opposite electrode 32, a streamer discharge like that shown in Fig. 2(d) occurs between the two electrodes 32 and 33. By doing so, cool plasma is generated in the discharge field when the streamer discharge occurs. Moreover, due to this cool plasma, radical species such as high speed electrons, ions, ozone, and hydroxy radical, and other excited molecules (excited oxygen molecules, excited nitrogen molecules, excited water molecules), and the like are generated. Furthermore, these active species flow with the air and are supplied to the titanium apatite support filter, and furthermore, supplied to the deodorizing agent 4a, thereby the performance of deodorization of the deodorizing agent 4a is reproduced.

Furthermore, these active species have extremely high energy level, and have the ability to deodorize and decompose small organic molecules, such as ammonias, aldehydes, nitrogen oxide, that are included in the air, even prior to reaching the titanium apatite support filter or the deodorizing agent 4a.

### (High Voltage Unit)

The high voltage unit 30 is connected to the streamer discharger 3a. The high voltage unit 30 can adjust the amount that the active species are generated by the streamer discharger 3a, by controlling the amount of discharge thereof (refer to Figs. 5 and 7).

### C. Deodorizing Agent Casing

The deodorizing agent casing 20 is made up of a main body portion 20a and a door 20b. The door 20b can be opened and closed and the deodorizing agent 4a can be taken out or put in from the opening of the deodorizing agent casing 20 when the door 20b is opened. A switch 51 of the streamer discharger is forced to be OFF when the door 20b is in the opened state, and switch 51 of the streamer discharger is ON when the door 20b is in the closed state. For this reason, the safety of a user is ensured.

### D. Photocatalytic Filter

A photocatalytic filter 42 is configured such that it is rolled up in the form of a roll having a length enough for a plurality of times of use, and when the surface thereof that is being used becomes dirty, the portion that is dirty is taken out and cut off. This photocatalytic filter is formed by gluing the electrostatic filter and the titanium apatite support filter together (not shown). The electrostatic filter adsorbs dusts and the like charged by the streamer discharger 3a. Dusts and the like that pass through the electrostatic filter are attached to the titanium apatite support filter. In addition, same as the prefilter, this titanium apatite support filter is formed from a PP fabric supporting the titanium apatite. The titanium apatite is an apatite, which a calcium atom of a portion of a calcium hydroxy apatite is substituted into a titanium atom by a method, such as ion exchange, and the like. This titanium apatite has a property of specifically adsorbing viruses, mold bacteria, bacteria, and the like that are contained in dusts, and the like. The photocatalytic function of this titanium apatite is activated by the active species provided from the streamer discharger 3a, and the titanium apatite inactivates or kills viruses, mold bacteria, bacteria, and the like.

### E. Plasma Catalytic Filter

Titanium dioxide of anatase type is supported on the plasma catalytic filter 43. The plasma catalytic filter 43 adsorbs viruses, bacteria, and the like, in the air, that were not adsorbed by the photocatalytic filter 42. In this plasma catalytic filter 43, the adsorbed bacteria, viruses, and the like are killed or inactivated by the titanium dioxide that is activated by active species.

### <Characteristics of the Second Embodiment>

(1)
   The deodorization function reproducing device 1a further includes the deodorizing agent 4a, which adsorbs at least one of either the odor molecules or harmful gas components floating in the air, and makes up the air purifier 10. In addition, using the deodorization function thereof, the air purifier 10 is also a deodorization function reproducing device. Consequently, this air purifier 10 can at least adsorb one of either the odor molecules or harmful gas components floating in the air, and deodorization is possible. Furthermore, since the deodorizing agent 4a, which needs to be changed in every predetermined period of time in the past, can be reused, it is possible to suppress the generation of waste, polluted water from washing it, and the like, and thereby can reduce burden to the environment when the reproducing process of the deodorizing agent 4a is performed. In addition, usability can be improved because reproducing can be done reliably in a short period of time with a simple operation.
(2)
   With this air purifier 10, the adjustment switch 52 can adjust the increase or decrease of the amount of active species generated by the streamer discharger 3a. The streamer discharger 3a increases the amount of the active species generated when the adjustment switch 52 is ON, and decreases the amount of active species generated when the adjustment switch 52 is OFF. Consequently, the amount of active species to be generated can be increased or decreased to only an amount for the reproducing process of the deodorizing agent 4a by turning the adjustment switch 52 ON or OFF, at a time when active species are used other than for the deodorizing agent 4a, and the like. By doing so, even if the adjustment switch 52 is OFF, the generation of the active species can be restrained without being terminated. As a result, it is possible to inhibit energy from being consumed meaninglessly.
   In addition, with this air purifier 10, it is possible to have the adjustment switch 52 be ON or OFF, by putting in or taking out the deodorizing agent 4a in or out of the deodorizing agent casing 20. Therefore, when the deodorizing agent 4a is not in the deodorizing agent casing 20, discharging meaninglessly can be prevented. For this reason, it is possible to inhibit energy from being consumed meaninglessly.

### <Third Embodiment>

### <Overall Configuration of an Air Conditioner>

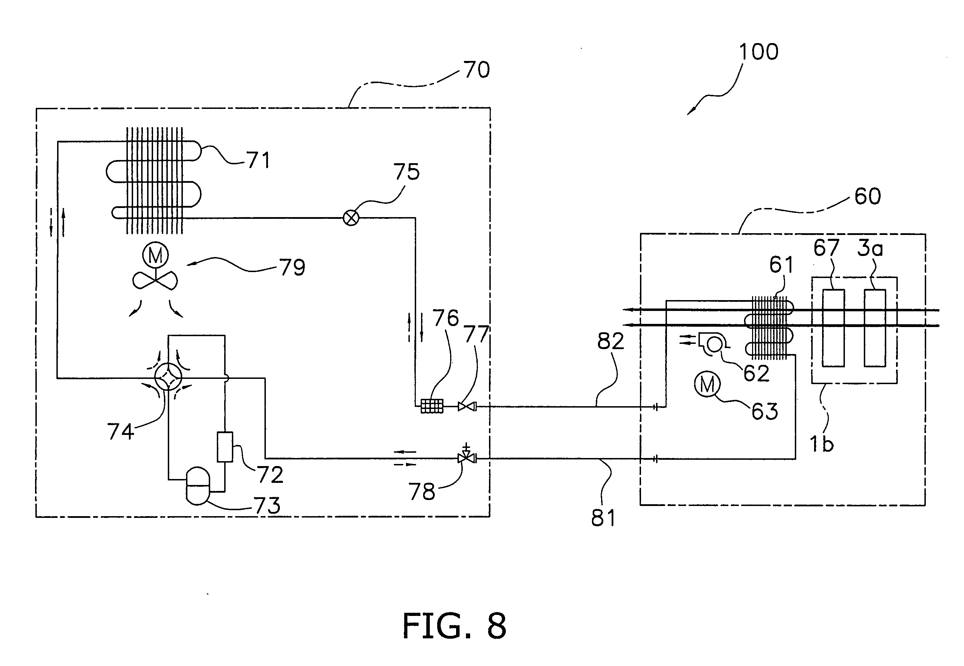

Fig. 8 is a system diagram of a refrigerant circuit of an air conditioner 100 in accordance with a third embodiment of the present invention, with the general flow of air added therein.

The refrigerant circuit of this air conditioner 100 is mainly made up of an indoor heat exchanger 61, an accumulator 72, a compressor 73, a four-way selector valve 74, an outdoor heat exchanger 71, and an electrical expansion valve 75.

The indoor heat exchanger 61, provided in the indoor device 60, performs heat exchange with the air in contact. The indoor heat exchanger 61 is composed of a heat transfer pipe that is folded back in a number of times at the two ends thereof in the longitudinal direction, and a plurality of fins penetrated by the heat transfer pipe, and performs heat exchange with the air that is in contact. In addition, in the interior of the indoor device 60, a cross-flow fan 62 (indoor blower portion), and an indoor fan motor 63 for driving the cross-flow fan 62 to rotate are provided. The cross-flow fan 62 is formed to be cylindrical, with a great number of blades provided on the circumferential surface thereof, and generates airflow in the direction of intersection with a rotational axis. This cross-flow fan 62 draws the indoor air inside the indoor device 60, and also blows out the air in which heat exchange has been done with the indoor heat exchanger 61 into the interior space.

A compressor 73, a four-way selector valve 74 connected to the compressor 73 at the discharging side, an accumulator 72 connected to the compressor 73 at the intake side, an outdoor heat exchanger 71 connected to the four-way selector valve 74, and an electrical expansion valve 75 connected to the outdoor heat exchanger 71 are provided in an outdoor device 70. The electrical expansion valve 75 is connected to a refrigerant liquid pipe 82 via a filter 76 and a liquid shut-off valve 77, and connected to one end of the indoor heat exchanger 60 through this refrigerant liquid pipe 82. Furthermore, the four-way selector valve 74 is connected to the a refrigerant gas pipe 81 via a gas shut-off valve 78, and connected to the other end of the indoor heat exchanger 61 through this refrigerant gas pipe 81. In addition, a propeller fan 79 for discharging air that has been heat exchanged at the outdoor heat exchanger 71 to the outside is provided in the outdoor device 70.

### <Configuration of the Indoor Device>

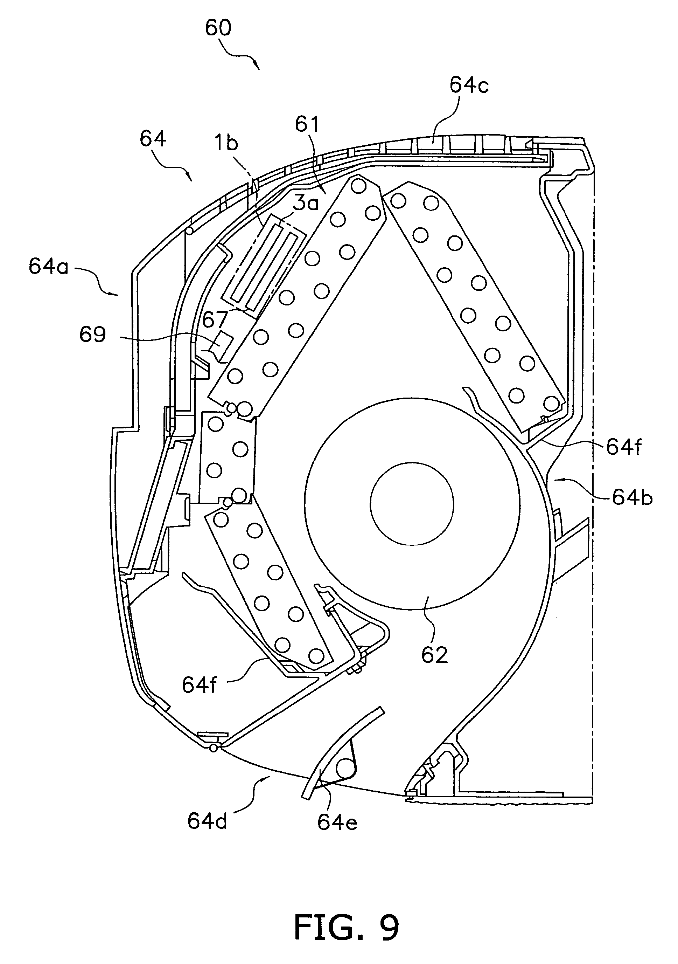

Next, the configuration of the indoor device 60 will be described based on a side section view of the indoor device 60 shown in Fig. 9.

The indoor device 60 includes an indoor device casing 64 that is long in the widthwise direction when viewed from the front, and the above described indoor heat exchanger 61 and the cross-flow fan 62 are accommodated inside the indoor device casing 64 of the indoor device 60. In addition, in the indoor device casing 64, a deodorization function reproducing device 1b and an indoor duct 69 are included.

### (1) Indoor Device Casing

The indoor device casing 64 is made up of a front panel 64a and a bottom frame 64b.

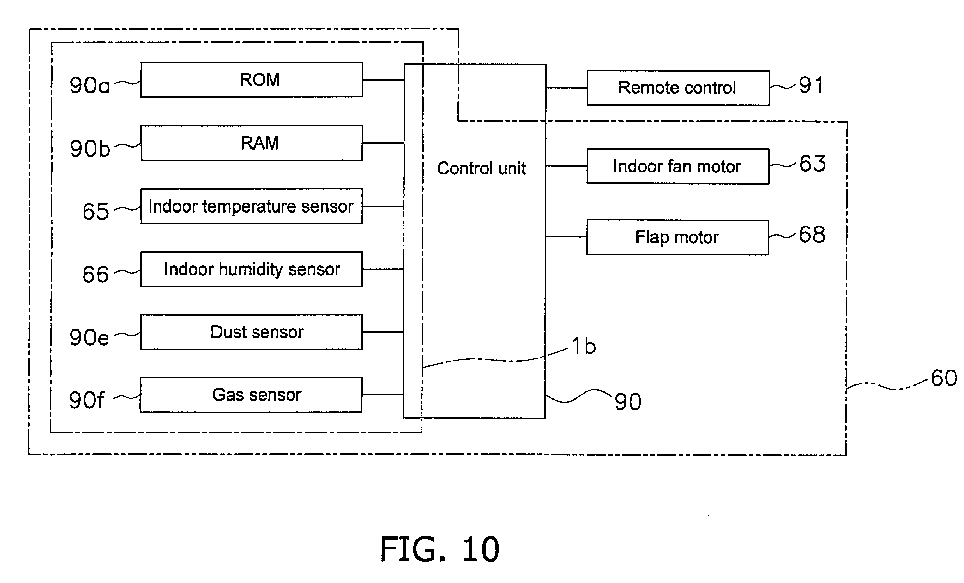

The front panel 64a covers the top, bottom, front, and the sides of the indoor device 60. The upper surface of the front panel 64a covers the top of the indoor heat exchanger 61, and on which a suction opening 64c composed of a plurality of slit shaped openings is provided. A blow out opening 64d composed of an opening along the longitudinal direction of the indoor device 60 is provided on the bottom surface of the front panel 64a. In addition, a horizontal flap 64e for adjusting the blow out angle of air blown into the interior space is provided on the blow out opening 64d. This horizontal flap 64e is provided rotatably with an axis parallel to the longitudinal direction of the indoor device 60 as the center thereof. The horizontal flap 64e can open and close the blow out opening 64d by being rotated by a flap motor 68 (refer to Fig. 10).

The bottom frame 64b forms the rear surface of the indoor device 60, and covers the rear side of the indoor heat exchanger 61.

In addition, a drain pan 64f for receiving water drops formed on the surface of the indoor heat exchanger 61 during heat exchange is provided below the indoor heat exchanger 61.

Furthermore, an indoor temperature sensor 65 for detecting the temperature of the indoor air drawn in from the suction opening 64c, and an indoor humidity sensor 66 for detecting the humidity of the indoor air are stored in the interior of the indoor device casing 64 (refer to Fig. 10).

### (2) Cross-Flow Fan

The cross-flow fan 62 is disposed roughly at the center of the indoor device 60 when viewed from the side. By being driven to rotate by the indoor fan motor 63 (refer to Fig. 10), the cross-flow fan 62 generates the flow of the air being drawn in from the suction opening 64c, passed through the indoor heat exchanger 61, and blown into the interior space from the blow out opening 64d.

### (3) Indoor Heat Exchanger

The indoor heat exchanger 61 is mounted to surround the front, top, and upper rear side of the cross-flow fan 62. The air drawn in from the suction opening 64c and the air sent in from the outdoor device 70 by a radial fan assembly (not shown) pass through to the side of the cross-flow fan 62 by being driven by the cross-flow fan 62, and the indoor heat exchanger 61 performs heat exchange with the refrigerant that passes inside the heat transfer pipe.

### (4) Deodorization Function Reproducing Device

The deodorization function reproducing device 1b is disposed in the interior of the indoor device casing 64, and deodorizes and eliminates bacteria in the air that passes nearby the deodorization function reproducing device 1b, during the time of an air discharging operation and an air circulating operation. More specifically, the deodorization function reproducing device 1b is disposed on an outer side of a second heat exchanging portion 61b, and disposed downstream of the suction opening 64c and upstream of the indoor heat exchanger 61, in the flow of air, which is being drawn in from the suction opening 64c and blown out from the blow out opening 64d. In addition, "outer side" referred here refers to a side opposite from an "inner side", which is the side in which the cross-flow fan 62 is disposed with respect to the indoor heat exchanger 61. The deodorization function reproducing device 1b is, for example, made up of a streamer discharger 3a having a needle-like discharging electrode and an opposite electrode that is disposed to be separated from the discharging electrode by only a very short distance, and a deodorizing agent casing 67 able to store a deodorizing agent 4b. The streamer discharger 3a makes streamer discharge occur by a discharge voltage being applied between the discharging electrode and the opposite electrode, and generates active species. Furthermore, the active speices decomposes odor molecules and harmful gas components and the like in the air that are adsorbed by the deodorizing agent 4b stored inside the deodorizing agent casing 67, to reproduce the deodorizing agent 4b. Moreover, the deodorization function reproducing device 1b deodorizes and eliminates bacteria in the air drawn in from the suction opening 64c and has not passed through the indoor heat exchanger 61. The air being deodorized and with bacteria eliminated passes through the indoor heat exchanger 61 and is blown into the interior space from the blow out opening 64d.

### (5) Indoor Duct

The indoor duct 69 is disposed inside the indoor device casing 64, and is provided along the surface of the indoor heat exchanger 61. The indoor duct 69 is connected to an air supply discharge duct at one end thereof, and an opening is provided on a portion facing the indoor heat exchanger 61 near the other end thereof, and on the tip of the other end thereof. Through the opening provided on the tip of the other end thereof, the indoor duct 69 is continuous with the interior space of the indoor device 60 that is positioned downstream of the suction opening 64c and upstream of the indoor heat exchanger 61 in the flow of air that is drawn in from the suction opening 64c and blown out from the blow out opening 64d.

### (6) Control Unit

The air conditioner 100 includes a control unit 90 as shown in Fig. 10. Various types of sensors, such as the indoor temperature sensor 65, indoor humidity sensor 66, dust sensor 90e, and gas sensor 90f are connected to the control unit 90, and the detected signals of each of the sensors are inputted thereto.

The dust sensor 90e can measure the particle concentration of dust and the like by irradiating the air that is brought in with light, and detecting the amount of light that is reflected diffusely by smoke, dusts, pollen, or other particles contained in the air, and reached the light receiving element. The gas sensor 90f can measure the gas concentration generated from harmful gas components, and odor molecules and the like decomposed by the active species.

In addition, by controlling each component of the indoor device 60 and the outdoor device 70 based on operation commands sent from a remote control 91, it is possible to change the operation mode of a deodorizing agent reproducing operation and deodorizing operation and the like, adjust the processing time of a reproducing operation, start or terminate an operation in the various operation modes, and so forth. In addition, Fig. 10 only shows the control unit of the indoor device 60.

### <Characteristics of the Third Embodiment>

This air conditioner 100 includes a deodorization function reproducing device 1b able to reproduce a deodorizing agent 4b that has been completely used, through active species that are generated by the streamer discharger 3b. Therefore, this air conditioner 100 can decompose the odor molecules and harmful gas components that are adsorbed in the deodorizing agent 4b, when the deodorizing agent 4b is inside the deodorizing agent casing. As a result, this can also contribute to an improvement in the deodorizing performance of this air conditioner 100.

### <Fourth Embodiment>

### <Overall Configuration of an Air Purifier>

### <Configuration of an Air Purifier 200>

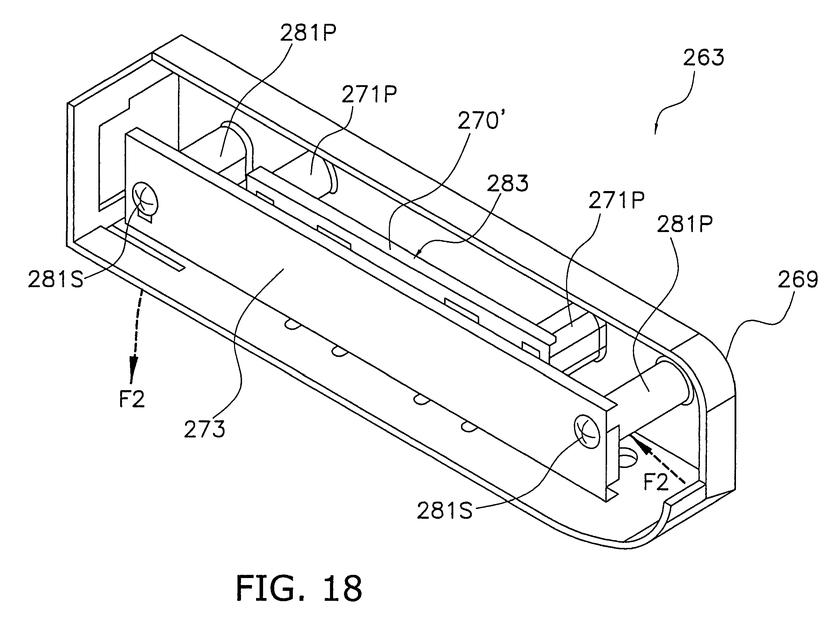

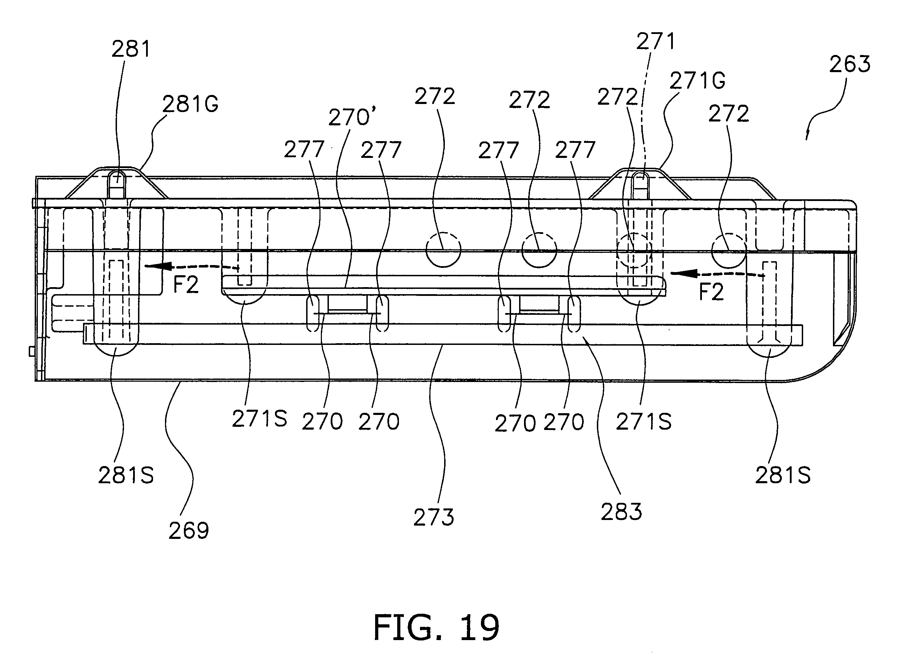

An air purifier 200 in accordance with a fourth embodiment of the present invention is shown in Fig. 1. This air purifier 200 is a floor type of air purifier that is set on the floor in an interior space, to keep the air in the interior space clean and make the interior space more comfortable. The air purifier 200 includes a main body portion 202 and a streamer discharging unit 263 (refer to Figs. 18 and 19). The main body portion 202 can be divided into a front portion and a rear portion, and a first main body portion 203 is provided on the rear side while a second main body portion 204 is provided on the front side. The streamer discharging unit 263 is positioned to extend in the front and rear direction on an upper right position in the main body portion 202 when viewed from the front side.

### <First Main Body Portion 203>

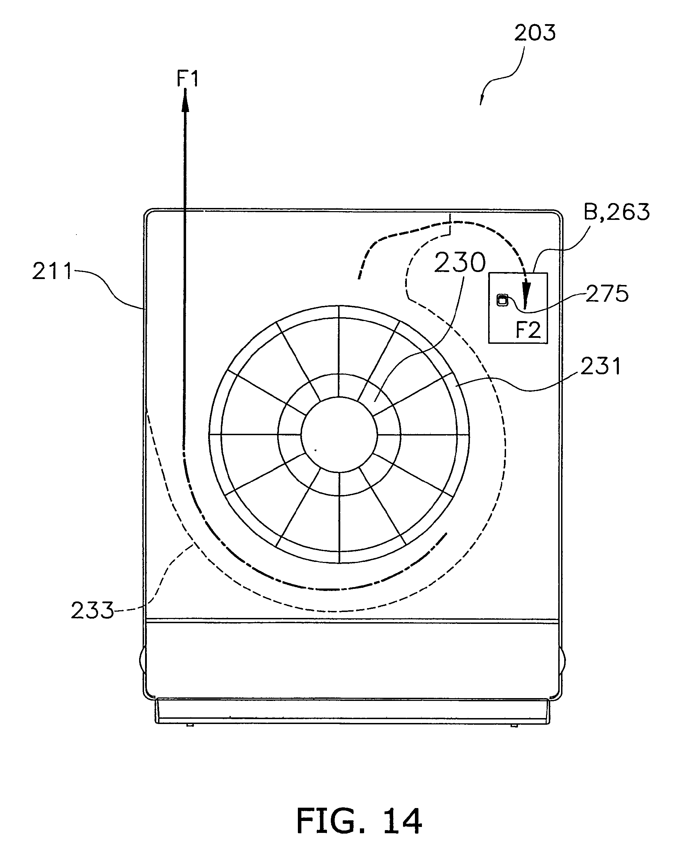

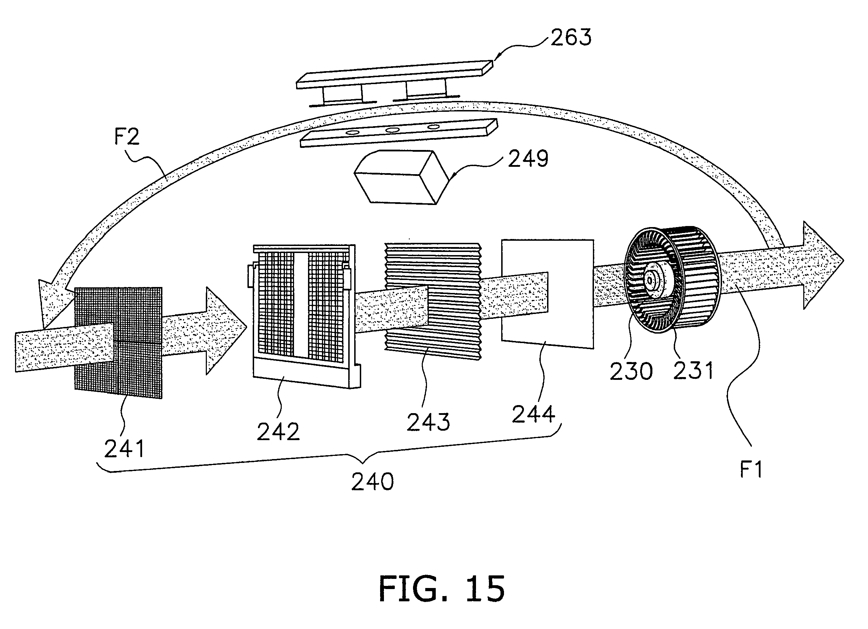

As shown in Fig. 14, the first main body portion 203 includes a first main body casing 211, a fan motor 230, a blower fan 231 (blower), a unit storage portion B, a photocatalytic filter 243 (refer to Fig. 15), and a plasma catalytic filter 244 (refer to Fig. 15). In addition, Fig. 14 is a front view of the first main body portion 203 in the state that the photocatalytic filter 243 and the plasma catalytic filter 244 are removed.

### <First Main Body Casing 211>

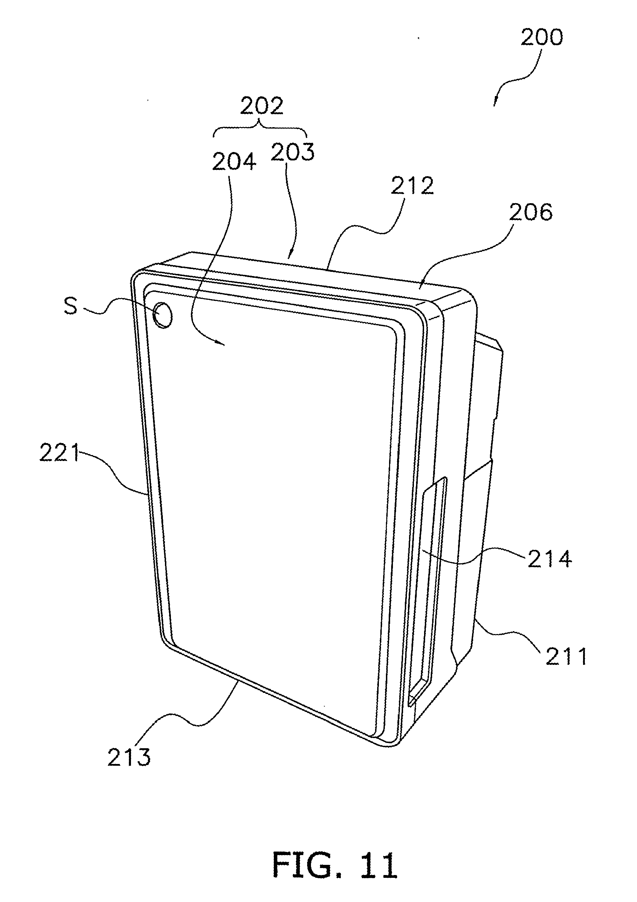

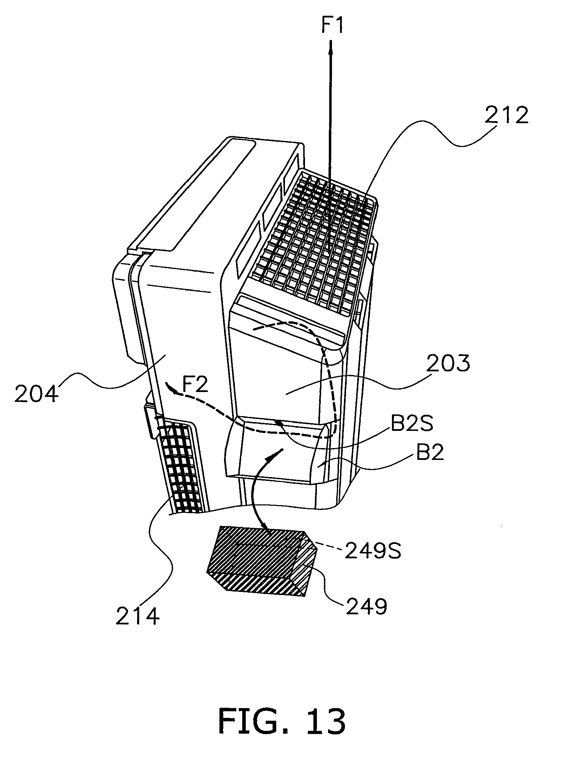

A first main body casing 211 has the fan motor 230, blower fan 231, unit storage portion B, photocatalytic filter 243, plasma catalytic filter 244, and the like stored in the interior thereof. As shown in Figs. 11 and 13, the first main body casing 211 includes a blow out opening 212, a lower suction opening 213, and side suction openings 214. The blow out opening 212 is provided on an end portion on the rear side of the upper portion of the first main body casing 211. The blow out opening 212 is an opening for blowing out cleaned air toward the top from the air purifier 200. The lower suction opening 213 and the side suction openings 214 are openings that are roughly rectangular, for drawing in air in the interior space inside the air purifier 200. The lower suction opening 213 is provided on an end portion on the front side below the first main body casing 211, same as the surface on which the blow out opening 212 is provided. The lateral length of the lower suction opening 213 is about the same as the lateral length of the front panel 221. The side suction openings 214 is a pair of openings provided on the front side of the left and right side portions of the first main body casing 211.

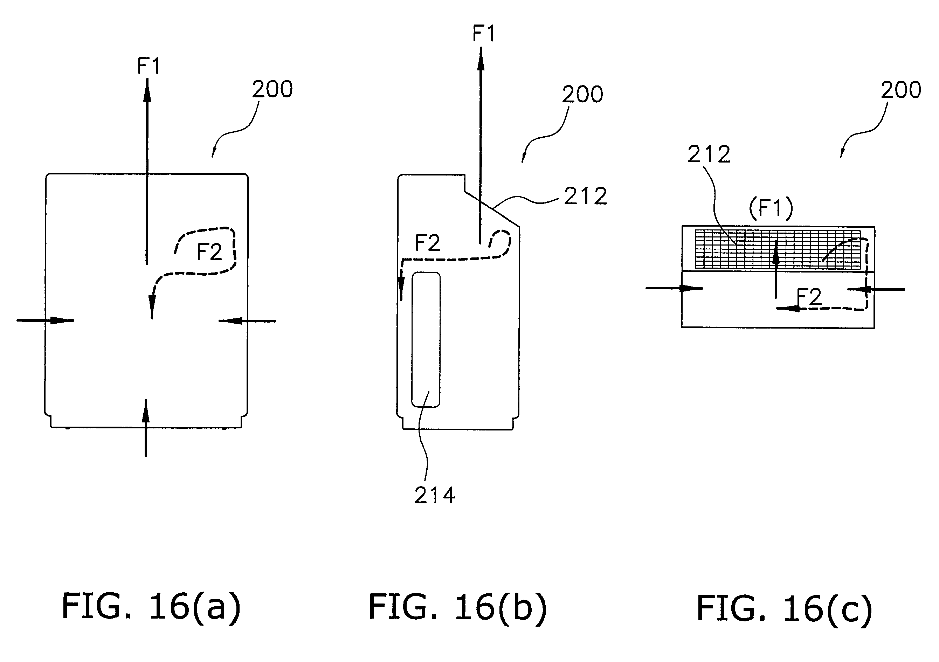

In addition, the first main body casing 211 forms the main body casing 206 with a front panel 221 to be described later. Inside the main body casing 206, a main airflow path through which air drawn in from the interior space through the suction opening 213 214 and 222 purified in an air purifying unit 240, and blown out into the interior space from the blow out opening 212 passes through, and a bypass airflow path near the blow out opening 212 for returning air to an upstream side are provided, and as shown in Figs. 13, 14, 16 (a), (b), and (c), a main airflow F1 flowing in this main airflow path and a bypass airflow F2 flowing in the bypass airflow path are generated by the blower fan 231. Here, Fig. 16(a) shows a front schematic diagram of the air purifier 200, Fig. 16(b) shows a right side schematic diagram of the air purifier 200, and Fig. 16(c) shows a top schematic diagram of the air purifier 200.

In addition, main airflow path basically flows from the front side toward the rear side, on a side more upstream than the blower fan 231. Hereinafter, "front side" means "an upstream side in the flowing direction of the main airflow of the main airflow path", and "rear side" means "a downstream side in the flowing direction of the main airflow of the main airflow path". That is, the bypass airflow flowing in the bypass airflow path is basically flowing from the rear side toward the front side.

Here, as shown in Figs. 13, 14, 16(a), (b), and (c), The main airflow F1 flowing in the main airflow path is an airflow for purifying the air drawn in from the lower suction opening 213 and the side suction openings 214 and blowing out the air from the blow out opening 212. In addition, the bypass airflow F2 flowing in the bypass airflow path flows to the upper side near the blow out opening 212, flows from the rear side toward the front side in the state that the streamer discharging unit 263 is stored in the unit storage portion B which stores the streamer discharging unit 263, and after flowing to the front near roughly the center in a plasma ion-wire 242 to be described later, it goes downward and is guided to the front side of a prefilter 241 to be described later.

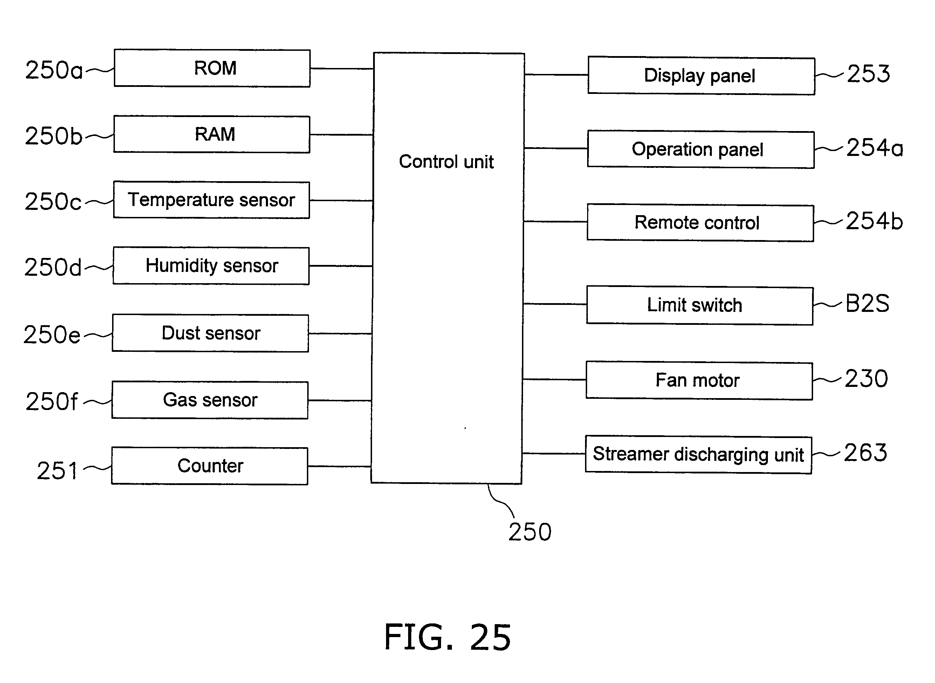

Here, as shown in Fig. 13, a cartridge storage portion B2 is provided on a side portion of the first main body portion 203 able to be opened or closed so that a deodorization catalytic cartridge 249 can be replaced, as this will be described later. Fig. 13 shows the "opened" state thereof. Here, a limit switch B2S is provided on the cartridge storage portion B2, and in the state that the deodorization catalytic cartridge 249 is stored in the cartridge storage portion B2, the positional relationship is configured so that the deodorization catalytic cartridge contacting portion 249S of the deodorization catalytic cartridge 249 is in contact with the limit switch B2S. By doing so, with the air purifier 200, as will be described later, in the state that the deodorization catalytic cartridge 249 is stored, the counting of a counter 251 (refer to Fig. 25) progresses. The replacement of the deodorization catalytic cartridge 249 can be informed after it is being used for a predetermined period of time. A reproducing process can be finished after it is used for a predetermined period of time.

### <Blower Fan 231 and Fan Motor 230>

The blower fan 231 and the fan motor 230 shown in Figs. 14 and 15 generate the main airflow F1 flowing in the main airflow path and the bypass airflow F2 flowing in the bypass airflow path. A centrifugal fan is adopted as the blower fan 231. Thus, the blower fan 231 draws in air from the rotational axis direction, and blows out air from the center of rotation outward in the radial direction. The fan motor 230 drives the blower fan 231 to rotate. An inverter motor with frequency controlled by an inverter circuit is adopted as the fan motor 230.

The air purifying unit 240 (refer to Fig. 15) to be described later is stored in a first space on the upstream side of the blower fan 231. In a second space on the downstream side of the blower fan 231, the fan motor 230, blower fan 231, and a side of the blower fan 231, in other words, a scroll 233 formed along the circumferential direction with the rotational axis of the blower fan as the center thereof are stored. Furthermore, air blown out from the blower fan 231 is sent into the indoor space from the blow out opening 212 along the scroll 233.

### <Photocatalytic Filter 243>

The photocatalytic filter 243, as shown in Fig. 15, is formed to be pleated, and is formed by gluing an electrostatic filter and a titanium apatite support filter together. In addition, this photocatalytic filter 243 is disposed so that the electrostatic filter faces the front side and the titanium apatite support filter faces the rear side. The electrostatic filter adsorbs dusts and the like charged when they pass through the plasma ion-wire 242 to be described later. The titanium apatite support filter adsorbs dusts and the like that passed through the electrostatic filter. Same as the prefilter 241, this titanium apatite support filter is formed from a PP fabric supporting titanium apatite. In addition, titanium apatite is apatite, which a calcium atom of a portion of a calcium hydroxy apatite is substituted into a titanium atom by a method, such as ion exchange, and the like. This titanium apatite has a property of specifically adsorbing viruses, mold bacteria, bacteria, and the like that are contained in dusts, and the like. The photocatalytic function of this titanium apatite is activated by the active species provided from the streamer discharging unit 263 to be described later, and the titanium apatite inactivates or kills viruses, mold bacteria, bacteria, and the like.

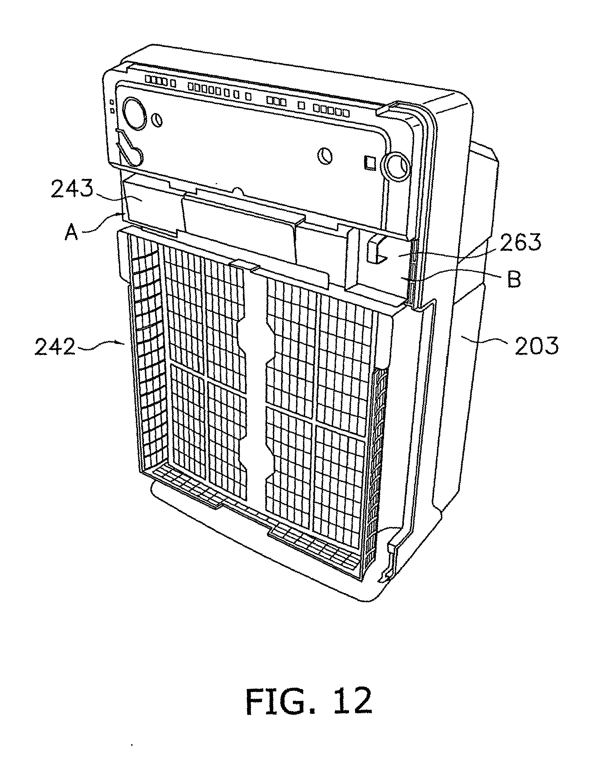

Furthermore, this photocatalytic filter 243 can be folded easily by folding along the folding lines, since it is formed to be pleated. For this reason, as shown in Fig. 12, the photocatalytic filter 243 in the folded state is stored in a replacement filter storage portion A that is formed on an upper portion.

### <Plasma Catalytic Filter 244>

The plasma catalytic filter 244 is disposed in front of the blower fan 231 and behind the photocatalytic filter 243. In other words, the plasma catalytic filter 244 is disposed between the blower fan 231 and the photocatalytic filter 243. Titanium dioxide of anatase type is supported on the plasma catalytic filter 244. Viruses, bacteria, and the like in the air that were not adsorbed on the photocatalytic filter 243 are adsorbed on the plasma catalytic filter 244. With this plasma catalytic filter 244, the adsorbed bacteria, viruses, and the like are inactivated or killed by titanium dioxide that is activated by active species.

### <Unit Storage Portion B>

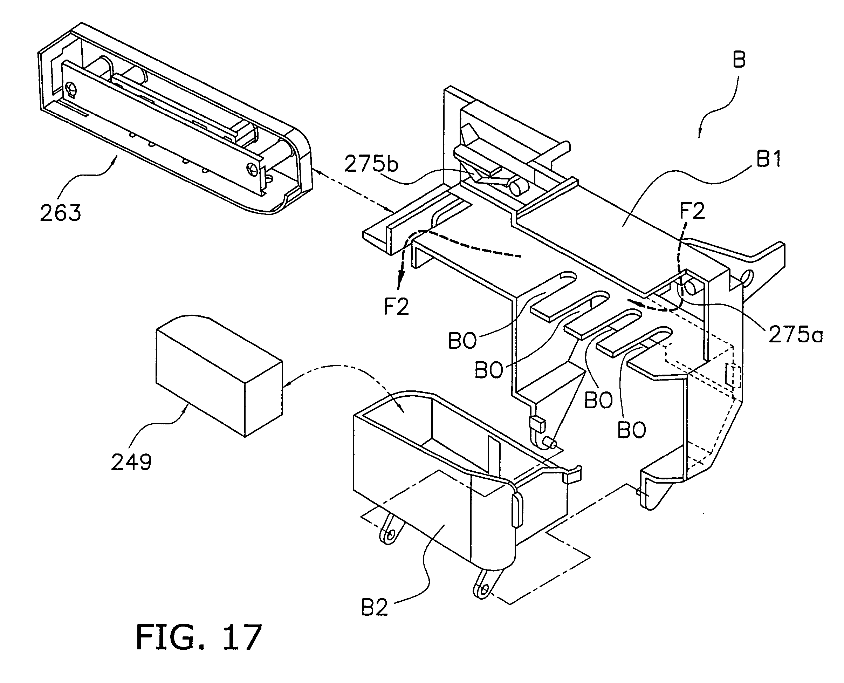

As shown in Fig. 17, the unit storage portion B forms a portion of the bypass airflow path through which the bypass airflow F2 flows, and in which the streamer discharging unit 263 is stored detachably. This unit storage portion B is made up of a unit storage main body B1, and a cartridge storage portion B2 mounted freely openable and closable with respect to the unit storage main body B1.

A deodorization catalytic cartridge 249 is stored in this cartridge storage portion B2. As shown in Fig. 17, in the state that the opening closing portion of this cartridge storage portion B2 is opened, the deodorization catalytic cartridge 249 can be replaced. In the state that the cartridge storage portion B2 is closed with the deodorization catalytic cartridge 249 stored, the cartridge storage portion B2 is provided with deodorization openings BO thereon so that air can flow back and forth into or out of the bypass airflow path of the unit storage main body B1. As a result, air flowing through the bypass airflow path is deodorized with the odor components being adsorbed and decomposed.

In addition, after this deodorization catalytic cartridge 249 is used for a certain period of time or longer, the effective absorption surface area thereof decreases, and the deodorizing effect thereof lowers. For this reason, it is necessary to perform a reproducing process decomposing odor components adsorbed on the surface thereof, or a process of replacing it is necessary when the life duration thereof ended.

Here, active species released from the streamer discharging unit 263 are supplied to the deodorization catalytic cartridge 249 from the bypass airflow path of the unit storage main body B1, through the deodorization openings BO (refer to Fig. 17) and four continuous holes 272 (refer to Fig. 19) to be described later. Thus, the attached odor components, being the reason that the deodorizing effect lowers, are decomposed by the active species. By doing so, the ability of the deodorization catalytic cartridge 249 to adsorb odor components is regained, and thereby it is reproduced to be able to be used as a deodorizing agent again.

In addition, even with a deodorization catalytic cartridge 249 that is reproduced in this way, there are limits to the way that it is able to effectively adsorb the odor components, and after being used continuously exceeding a certain period of time, even if it is reproduced through the active species, the lowering of deodorizing ability still cannot be stopped. Consequently, here, as shown in Fig. 13, a limit switch B2S is provided on the unit storage portion B, and the limit switch B2S is structured to be pressed by the deodorization catalytic cartridge contacting portion 249S in the state that the deodorization catalytic cartridge 249 is stored in the unit storage portion B. As shown in Fig. 25, a counter 251 that counts only in the state that the deodorization catalytic cartridge 249 is stored in the unit storage portion B and the limit switch B2S is ON is provided in the air purifier 200 to be connected to the control unit 250. As a result, the period of time that the deodorization catalytic cartridge 249 is stored and being used can be known, and the time for replacing it can be known, through the control unit 250 (refer to Fig. 25) judging whether or not a predetermined time has been exceed.

Main body contacts 275a and 275b are provided on the unit storage main body B1, so that it is electrically connected and in a conducted state, in the state that the streamer discharging unit 263 is mounted. The main body contacts 275a and 275b are formed from a metal plate, and are connected to the power source via a power circuit (not shown) and a power cord (not shown) stored in the first main body portion 203. As a result, the main body contacts 275a and 275b are provided to be separated from each other in the longitudinal direction and the vertical direction, and transfer electric current from the power source to the side of the streamer discharging unit 263, by being in contact with a discharging unit contact 271 to be described later. Here, the main body contact 275a is positioned to the rear side and is in contact with the streamer discharging electrode 270, and the main body contact 275b is positioned to the front side and is in contact with an earth plate 273. Furthermore, the unit storage main body B1 is formed by an insulating material, such as resin and the like.

### <Streamer Discharging Unit 263>

As described above, the streamer discharging unit 263 is disposed to be stored in the unit storage portion B on the upper right of the first main body portion 203, and forms a portion of the main airflow path (refer to Figs. 12 and 14).

The streamer discharging unit 263, as shown in Figs. 18 and 19, includes a discharging unit casing 269, a discharging portion 283, and discharging unit contacts 271 and 281. By generating streamer discharge, the streamer discharging unit 263 generates active species to be provided to the photocatalytic filter 243 and releases the active species in the bypass airflow F2.

### <Discharging Unit Casing 269>

The discharging portion 283 is installed in the discharging unit casing 269. The discharging unit casing 269 is a box shaped member formed from resin, and has an outer shape that matches the unit storage portion B. The discharging unit casing 269 is open on the upper surface and right side surface, and covers the front side, rear side, left side, and bottom of the discharging portion 283.

The discharging unit casing 269, as shown in Fig. 17, is inserted inside the unit storage portion B, and mounted attachably and detachably in the unit storage portion B. The discharging unit casing 269 covers around the discharging portion 283 and forms the bypass airflow path together with the unit storage portion B, by being mounted in the unit storage portion B.

Contact covers 271G and 281G are provided at two locations and are separated from each other in the longitudinal direction and vertical direction, on the left side of the rear surface of the discharging unit casing 269 (upper side shown in Fig. 19). The contact covers 271G and 281G have a function for guiding the insertion of the streamer discharging unit 263 in the unit storage portion B at the time of insertion, in the front and rear direction, by sliding against a member on the side of the unit storage portion B.

As shown in Fig. 19, the discharging unit contacts 271 and 281 are provided to separate in the longitudinal direction and vertical direction and positioned corresponding to the positions of the contact covers 271G and 281G respectively. In the state that the streamer discharging unit 263 is mounted, the left side shown in Fig. 19 is the front side, and the right side is the rear side of the air purifier 200. As described above, while the discharging unit contact 271 and the discharging unit contact 281 are being guided by the contact covers 271G and 281G, they are in contact with the main body contact 275a and main body contact 275b respectively, in the state that the streamer discharging unit 263 is inserted in the unit storage portion B.

In addition, a plurality of minute holes 277 is provided on an intermediate portion in the longitudinal direction on the bottom of the discharging unit casing 269. Furthermore, four continuous holes 272 are provided on a rear portion in the longitudinal direction on the bottom of the discharging unit casing 269, opening to the bottom, and positioned corresponding to the four deodorization openings BO of the above described unit storage portion B, when being stored.

### <Discharging Portion 283>

The discharging portion 283 is a main portion for generating the streamer discharge, and has streamer discharging electrodes 270 and an earth plate 273.

### (Streamer Discharging Electrodes 270)

The streamer discharging electrodes 270 are made up of terminals mounted to portions of a metal plate 270' that are cut and bent outward, and by applying discharge voltage, streamer discharge occurs between the streamer discharging electrodes 270 and the earth plate 273. The streamer discharging electrodes 270 and the metal plate 270' are screwed onto the discharging unit casing 269 via two insulating supporting columns 271P by screws 271S (two) that pass through inside the insulating supporting columns 271P. One of the two insulating supporting columns 271P is screwed to be in contact with the discharging unit contact 271. As a result, the streamer discharging unit 263 is mounted to the unit storage portion B, and in the state that the discharging unit contact 271 is in contact with the main body contact 275a, the discharging unit contact 271 is in a state of being electrically connected to the streamer discharging electrodes 270 via the screw 271S. For this reason, the discharging unit contact 271 can transfer discharge voltage from the main body contact 275a to the streamer discharging electrodes 270.

### (Earth Plate 273)

The earth plate 273 is formed from a metal plate, and has roughly a rectangular outer shape bigger than the metal plate 270' of the streamer discharging electrodes 270. The earth plate 273 is disposed parallel to the streamer discharging electrodes 270, and disposed separately near the streamer discharging electrodes 270. The earth plate 273 is screwed onto the discharging unit casing 269 via two insulating supporting columns 281P by screws 281S (two) that pass through inside the insulting supporting columns 281P. One of the two insulting supporting columns 281P is screwed to be in contact with the discharging unit contact 281. As a result, the streamer discharging unit 263 is mounted to the unit storage portion B, and in the state that the discharging unit contact 281 is in contact with the main body contact 275b, the discharging unit contact 281 is in a state of being electrically connected to the earth plate 273 via the screw 281S.

As described above, the main body contact 275a corresponding to the streamer discharging electrodes 270 is positioned on the rear side of the main body, thereby it is difficult for a user to reach it with his or her hand, and this ensures the safely of a user. In addition, the streamer discharging electrodes 270 and the earth plate 273 are fixed to the discharging unit casing 269 that is formed by resin via the insulating supporting columns 271P and 281P respectively, thereby insulation is ensured.

Furthermore, in the above described configuration, the discharging unit casing 269 divides the space in which the streamer discharging electrodes 270 and the earth plate 273 are disposed, and the space in which the contact points of the discharging unit contacts 271 and 281 are disposed. As a result, the contact points of the discharging unit contacts 271 and 281 are arranged in spaces that are divided off from the space in which the streamer discharging electrodes 270 and the earth plate 273 are disposed.

By doing so, the active species generated from the streamer discharging electrodes 270 are released to the bypass airflow flowing in the bypass airflow path. The active species released in this streamer discharging unit 263 flow through an upper guide 242G and a front guide 242H of the plasma ion-wire 242 to be described later, and are provided to the front side of the prefilter 241.

### <Second Main Body Portion 204>

As shown in Figs. 11, 12, and 15, the second main body portion 204 is mounted attachably and detachably on the front of the first main body portion 203. The second main body portion 204 includes a front panel 221, the prefilter 241 (refer to Fig. 15), and the plasma ion-wire 242 (refer to Fig. 15).

### <Front Panel 221>

The front panel 221 is provided on the front of the second main body portion 204. The height of this front panel 221 is configured to be longer than the depth of the air purifier 200. In addition, in this case, a semitransparent portion S is provided so that it is possible to detect movements of human beings through a human detection sensor (not shown) used for controlling the ON and OFF, and the like.

### <Prefilter 241>

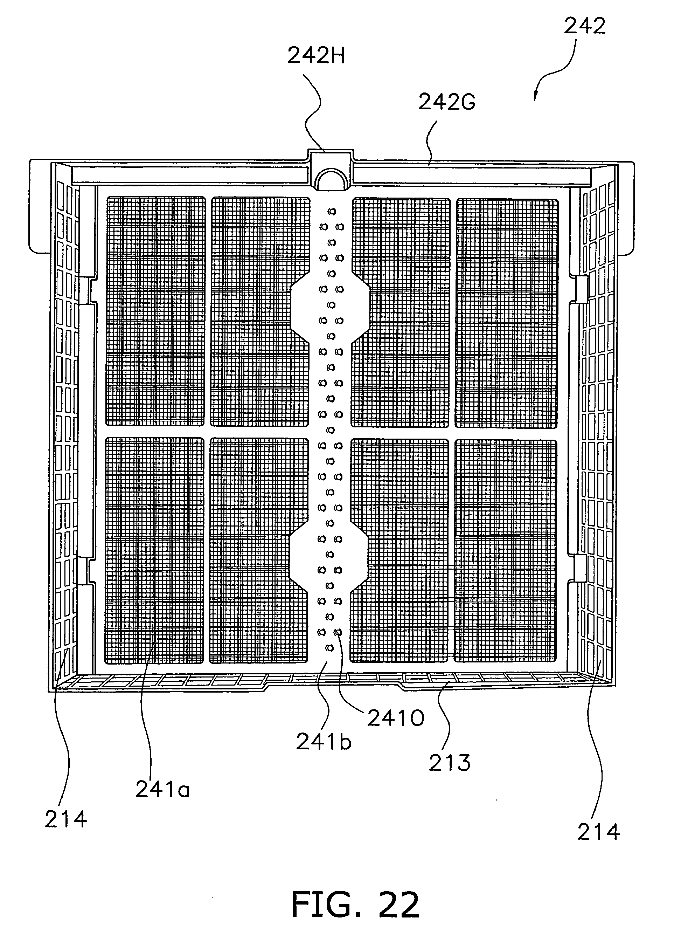

As shown in Figs. 15 and 22, the prefilter 241 is provided with a filter portion 241a and a divider ventilation portion 241b.

The filter portion 241a is provided on the rear side of the front panel 221, and removes comparative large dusts and dirt. The filter portion 241a is made up of a net that is a threadlike resin net made of polypropylene (hereinafter referred to as PP), and a frame for holding the net. A catechin and photocatalyst of visible light type are supported, to be exposed on the air side, on the fabric that forms the net of the filter portion 241a. The photocatalyst of visible light type includes oxidized titanium and the like that a photocatalytic effect is activated by visible light, and removes bacteria and viruses of mold bacteria, bacteria, and the like contained in dusts and the like that are attached to the filter portion 241a. In addition, catechin is a type of polyphenol, and is the generic name of epicatechin, epigallocatechin, epicatechin gallate, epigallocatechin gallate, and the like. This catechin suppresses the multiplication of bacteria of mold bacteria, bacteria, and the like contained in dusts and the like attached on the filter portion 241a, and inactivates viruses.

As shown in Fig. 22, the divider ventilation portion 241b is provided to divide the filter portion 241a on the left and right, going across the top and bottom in the center of the prefilter 241. This divider ventilation portion 241b has a plurality of holes 2410 that pass through in the front and rear direction.

### <Plasma ion-wire 242>

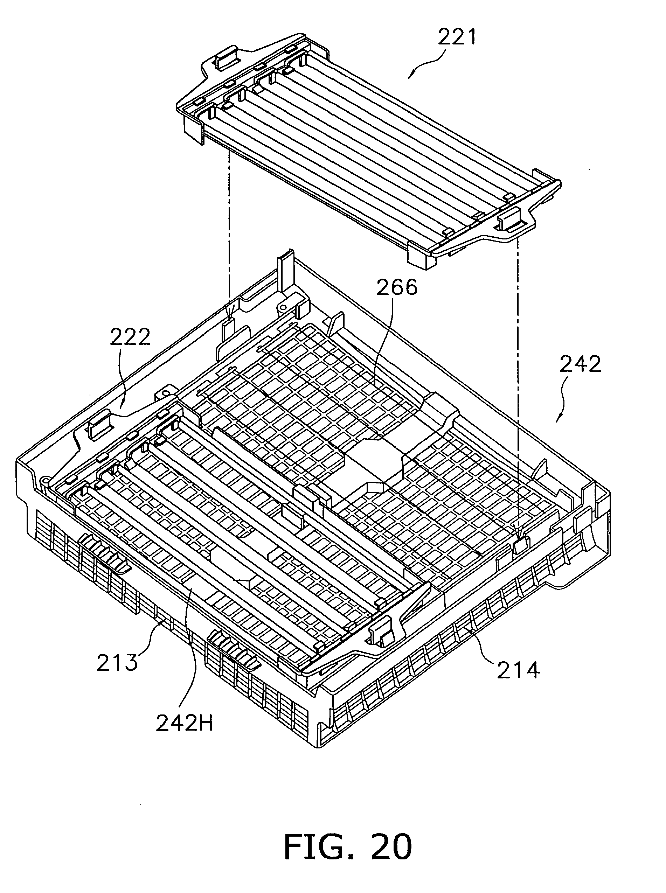

As shown in Fig. 11, the plasma ion-wire 242 is provided behind the prefilter 241, and is provided on the rear side of the second main body portion 204. The plasma ion-wire 242 charges comparatively small dusts floating in the air that passed through the prefilter 241. As shown in Fig. 20, the plasma ion-wire 242 mainly includes a pair of opposite electrodes 221 and 222, and a plurality of ion-wires 266 (linear electrode portions). In addition, in Fig. 20, only a part of the ion-wires 266 of the plurality of ion-wires 266 is numbered, and the others are omitted.

### (Opposite Electrodes 221, 222)

As shown in Fig. 20, the pair of opposite electrodes 221 and 222 is metal plates having a section in a square corrugated shape, and is mounted so as to separate at the top and bottom. The opposite electrodes 221 and 222 are disposed near the ion-wires 226, and generate corona discharge between the ion-wires 266, through high voltage electric current flowing through the ion-wires 266.

### (Ion-wires 266)

The plurality of ion-wires 266 are provided for charging comparatively small dusts floating in the air that passed through the prefilter 241, together with the opposite electrodes 221 and 222. The ion-wires 266 are disposed in front of the opposite electrodes 221 and 222. By applying discharge voltage to the ion-wires 266, corona discharge occurs between the ion-wires 266 and the opposite electrodes 221, 222. The ion-wires 266 are disposed so as to intersect with the air pathway, and disposed in the airflow that passes through the air pathway. In addition, the ion-wires 266 are provided parallel to the vertical direction, and a plurality of ion-wires 266 is disposed with a plurality of them lined up in the horizontal direction. These ion-wires 266 are formed by tungsten wire rod and the like of very small diameter, and are used as discharging electrodes for charging dusts and the like.

### (Upper Guide 242G, Front Guide 242H)

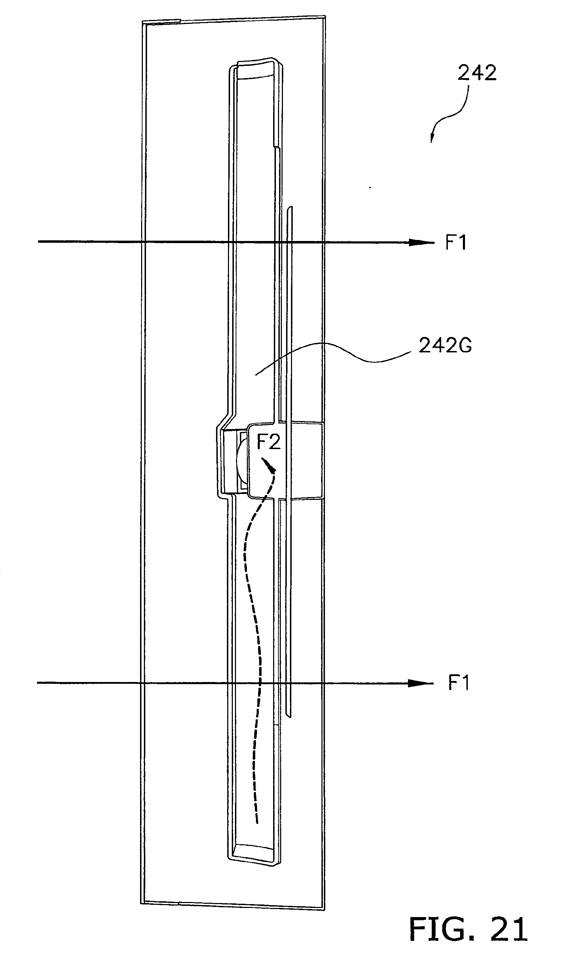

As shown in Fig. 21, the upper guide 242G, which is a groove sunken downwardly and long in the left and right direction, is formed on the top of the plasma ion-wire 242. By mounting the plasma ion-wire 242 to the first main body portion 203, this upper guide 242G is covered at the top by the first main body portion 203, and this upper guide 242G forms a part of the bypass airflow path. The bypass airflow F2 containing active species flows from the opening portion on the front side of the above described unit storage portion B to the right side of this upper guide 242G. Furthermore, the bypass airflow F2 flowing in this manner flows downward from near the center of the upper guide 242G.

In addition, as shown in Figs. 20, 23 and 24, near the center of the front of the plasma ion-wire 242, the front guide 242H is provided, which is made up of a rib 243R protruded to the front side on the edge and groove arranged sunken to the rear side across the top and bottom. This front guide 242H has a shape corresponding to the outline of the divider ventilation portion 241b of the above described prefilter 241. As shown in Fig. 22, the underside surface of the prefilter 241 is in contact with the rib 243R of the plasma ion-wire 242, and the bypass airflow path is formed between the divider ventilation portion 241b of the prefilter 241 and the front guide 242H, and the bypass airflow from the upper guide 242G flows downwardly.

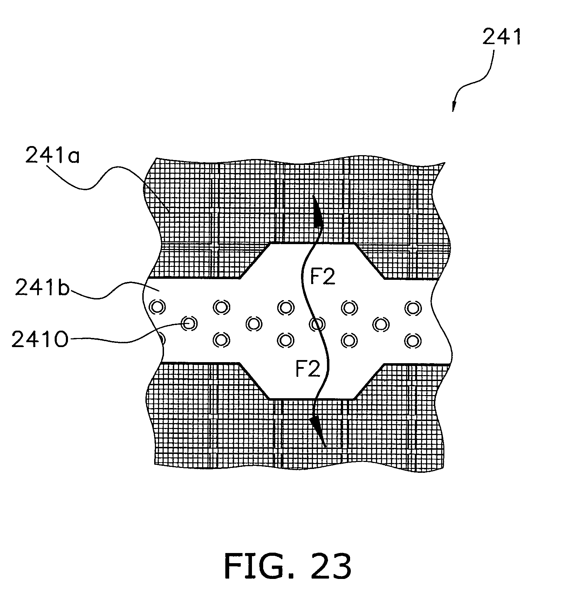

At this time, through the air that contains active species that passes through the front guide 242H, as shown in Fig. 23, the bypass airflow F2 is released toward the front side of the filter portion 241a through the holes 241O of the divider ventilation portion 241b.

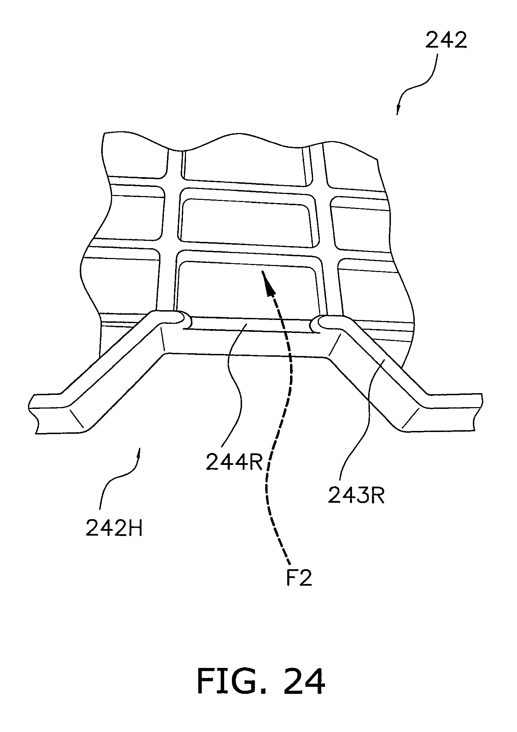

In addition, as shown in Figs. 23 and 24, a depressed portion 244R sunken toward the rear side is provided on the portion of the rib 243R provided on the plasma ion-wire 242 that corresponds to a part of a bulging portion of the divider ventilation portion 241b. As a result, a part of the bypass airflow F2 containing the active species is also provided to the downstream side of the prefilter 241, and active species can also be sufficiently provided to a cleaning portion on the back of the prefilter 241.

### <Control Unit 250>

As shown in Fig. 25, a ROM 250a in which various parameters such as odor component allowable amount data, replacement predetermined time data of the deodorization catalytic cartridge 249, and a control program are stored, and a RAM 250b in which variables and the like in process are temporarily stored, and the like are connected to the control unit 250 provided in the air purifier 200. In addition, a temperature sensor 250c, a humidity sensor 250d, a dust sensor 250e, a gas sensor 250f, a limit switch B2S, a counter 251, a display panel 253, an operation panel 254a, a remote control 254b, a fan motor 230, and a streamer discharging unit 263 are all connected to the control unit 250.

The detected signals of each of the sensors, of the various types of the sensors of the temperature sensor 250c, humidity sensor 250d, dust sensor 250e, and the gas sensor 250f, are outputted to the control unit 250. The dust sensor 250e can measure the particle concentration of dust and the like by irradiating the air that is brought in with light, and detect the amount of light that is shot randomly by smoke, dusts, pollen, or other particles contained in the air, and reached the light receiving element. The gas sensor 250f can measure the gas concentration generated from harmful gas components, and odor molecules and the like decomposed by the active species. Through these various types of sensors, the reproducing status of the deodorization catalytic cartridge 249 is evaluated and the reproducing time can be automatically adjusted.

The switch of the limit switch B2S becomes ON when it is being pressed by the deodorization catalytic cartridge contacting portion 249S in the state that the deodorization catalytic cartridge 249 is stored in the cartridge storage portion B2 of the unit storage portion B, and the switch of the limit switch B2S becomes OFF when the deodorization catalytic cartridge contacting portion 249S is away from it in the state that the deodorization catalytic cartridge 249 is not being stored. The control unit 250 makes the counter 251 proceed with counting when the state of the switch of the limit switch B2S is ON, and makes the counter 251 stop counting when the state of the switch of the limit switch B2S is OFF. As a result, the control unit 250 performs a process of judging whether or not the counting by the counter 251 exceeded the number of times in the replacement predetermined time data of the deodorization catalytic cartridge 249 stored in the ROM 250a, and if the control unit 250 judged that it has exceeded a predetermined number of times, the control unit 250 performs a process of informing from the display panel 253 a sign for informing the replacement of the deodorization catalytic cartridge 249.

Furthermore, in the state that the switch of the limit switch B2S is ON, the control unit 250 makes the display panel 253 display that the deodorization catalytic cartridge 249 is being reproduced, until the amount of the odor components detected by the gas sensor 250f described above falls below that of the odor component allowable amount data stored in the ROM 250a. In addition, the configuration may be a simple configuration by having the reproducing process here performed for only a predetermined time set in advance by a timer and the like, and also having the displaying performed for only a predetermined time accordingly.

In addition, other than the above described kind of display, the display panel 253 also displays the operation mode, monitor information through the various sensors 250c to 250f, timer information, maintenance information, and the like. In addition, this display panel 253 can be made up of a liquid crystal display panel, LED, some other display device, or a combination of these.

A user can reset the counting of the counter 251 by inputting that the deodorization catalytic cartridge 249 has been replaced with a new one through the operation panel 254a and the remote control 254b, if the user judges through the replacement notification from the display panel 253 and replaces the deodorization catalytic cartridge 249 with a new one. As a result, the counting of the total usage time of the replaced new deodorization catalytic cartridge 249 can be started from the beginning again. In addition, a user can change the operation mode of deodorizing agent reproducing operation and deodorizing operation and the like, adjust the processing time of reproducing operation, start or terminate an operation in the various operation modes, and so forth, through the operation panel 254a and the remote control 254b.

Furthermore, the fan motor 230 and the streamer discharging unit 263 are connected to the control unit 250. The control unit 250 can control the air volume through the fan motor 230 and the discharge amount related to the generation of active species through the streamer discharging unit 263, according to the detected results of the various sensors 250c to 250f and the operation of the user and the like.

### <Air Purification Effect through the Air Purifying Unit 240>

As shown in Fig. 15, this air purifier 200 includes the streamer discharging unit 263, the deodorization catalytic cartridge 249, and the air purifying unit 240 (air purifying portion) that includes the prefilter 241, the plasma ion-wire 242, the photocatalytic filter 243, and the plasma catalytic filter 244. The air purifier 200 purifies air by removing foreign substances contained in the indoor air drawn in from each of the suction openings (lower suction opening 213, side suction openings 214). The air purification effect through the air purifying unit 240 will be described below.

Interior air drawn in from the lower suction opening 213 and the side suction openings 214 first passes through the filter portion 241a of the prefilter 241. At this time, comparatively large dusts and dirt are removed from the air. In addition, the multiplication of viruses and bacteria such as mold bacteria, bacteria, and the like contained in the dusts and the like attached on the filter portion 241a of the prefilter 241 is suppressed and viruses are inactivated by the effect of catechin and photocatalyst included in the prefilter 241.

Airflow that passed through the prefilter 241 passes through between the ion-wires 266 and the opposite electrodes 221 and 222. Discharge occurs between the ion-wires 266 and the opposite electrodes 221 and 222 when high voltage is applied between the ion-wires 266 and the opposite electrodes 221 and 222. As a result, dusts and the like contained in the airflow that passes through between the ion-wires 266 and the opposite electrodes 221 and 222 are charged positively.

The airflow that passed through between the ion-wires 266 and the opposite electrodes 221 and 222 passes through the photocatalytic filter 243. At this time, dusts and the like that are charged at the time of passing through the plasma ion-wire 242 are adsorbed by the electrostatic filter. In addition, dusts and the like that passed through the electrostatic filter are adsorbed by the titanium apatite support filter. Furthermore, since viruses, bacteria, and the like contained in dusts are also charged when they passed through the ion-wires 266 and the opposite electrodes 221 and 222, the absorption efficiency of viruses and bacteria to the titanium apatite improves.

The airflow that passed through the photocatalytic filter 243 passes through the plasma catalytic filter 244. Viruses, bacteria, and the like in the air that were not adsorbed onto the photocatalytic filter 243 are adsorbed on the plasma catalytic filter 244.

The purified main airflow F1 that passed through the plasma catalytic filter 244 is blown out into the interior space from the blow out opening 212. In addition, a part of the air that passed through the plasma catalytic filter 244 is not blown out into the interior space, but becomes the bypass airflow F2 and flows toward the bypass airflow path.

As described above, the streamer discharging unit 263 and the deodorization catalytic cartridge 249 are provided in the bypass airflow path. In the streamer discharging unit 263, direction current, alternate current, or pulsed discharge voltage is applied between the streamer discharging electrodes 270 and the earth plate 273, and streamer discharge occurs between the streamer discharging electrodes 270 and the earth plate 273. When streamer discharge occurs, cool plasma is generated in the discharge field, and active species are generated inside the discharging unit casing 269 and released into the bypass airflow F2. In addition, these active species have extremely high energy level, and have the ability to deodorize and decompose small organic molecules such as nitrogen oxide, aldehydes, ammonias that are contained in the air, even before they reach the photocatalytic filter 243.

Here, air that passes through near the streamer discharging electrodes 270 is once purified by the prefilter 241, plasma ion-wire 242, photocatalytic filter 243, and the plasma catalytic filter 244. For this reason, it is possible to prevent ammonium nitrate and the like from attaching onto and dust from collecting on a terminal portion of the streamer discharging electrodes 270. As a result, streamer discharge can be generated stably.

As described above, active species generated from this streamer discharging unit 263 are provided to the deodorization catalytic cartridge 249 through the four continuous holes 272 and the deodorization openings BO (refer to Figs. 17 and 19), and the deodorization catalytic cartridge 249 is reproduced. More specifically, odor components adsorbed on the surface of the deodorization catalytic cartridge 249 are decomposed, and the ability of adsorbing odor components is improved.

In this manner, the bypass airflow F2 having the active species passes through the bypass airflow path, and is provided to the front side and rear side of the prefilter 241, and purification can be done by the effect thereof on dusts and the like that are attached on the prefilter 241.

Air containing the active species passes through between the ion-wires 266 and the opposite electrodes 221 and 222, and passes through the photocatalytic filter 243. At this time, dusts and the like that are charged when passing through the plasma ion-wire 242 are adsorbed by the electrostatic filter. Furthermore, at the time that dusts and the like that passed through the electrostatic filter are adsorbed by the titanium apatite support filter, the photocatalytic function of titanium apatite is activated by active species provided from the streamer discharging unit 263, and viruses, mold bacteria, bacteria, and the like can be killed or inactivated.

Furthermore, viruses, bacteria, and the like in the air that were not adsorbed onto the photocatalytic filter 243 are adsorbed by the plasma catalytic filter 244, and these bacteria, viruses, and the like are killed or inactivated by titanium dioxide that is activated by air containing the active species.

### <Characteristics of the Fourth Embodiment>

This air purifier 200 has a configuration in which the deodorization catalytic cartridge 249 is spatially continuous with the bypass airflow path, through the four continuous holes 272 and the deodorization openings BO. For this reason, active species generated in the streamer discharging unit 263 are provided to the deodorization catalytic cartridge 249 through these four continuous holes 272 and the deodorization openings BO, and the adsorbed odor components are decomposed, and reproduction can be done. In addition, during reproduction, since the limit switch B2S is ON, the control unit 250 displays that reproduction is in process on the display panel 253, and thereby a user will be able to know that reproduction is being done.

In addition, with this air purifier 200, while the control unit 250 refers to count data through the counter 251, the control unit 250 can inform the user of the time for replacing the deodorization catalytic cartridge 249 by displaying it on the display panel 253. The counter 251 does not simply count the operation time of the air purifier 200, but only counts when the deodorization catalytic cartridge 249 is actually stored in the cartridge storage portion B2 and that the state of the limit switch B2S is ON. For this reason, a user will be able to know the more precise time for replacing the deodorization catalytic cartridge 249.

In addition, with this air purifier 200, the bypass airflow that passes through the streamer discharging unit 263 is purified in the main airflow path. Accordingly, unclean substances that exist in the air can be prevented from attaching onto the discharging portion 283. For this reason, plasma discharge can be done stably. As a result, the air purification effect can be stabilized because active species can be provided stably to the air purifying unit 240.

### <Modified Examples>

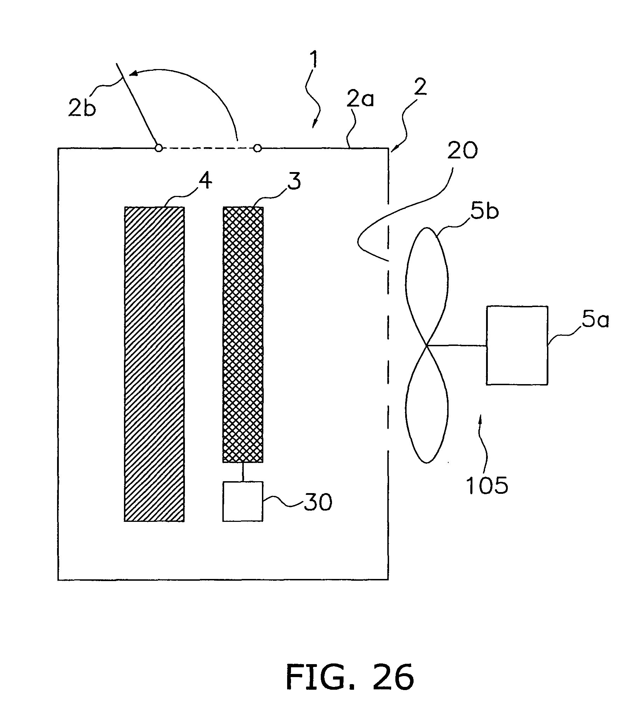

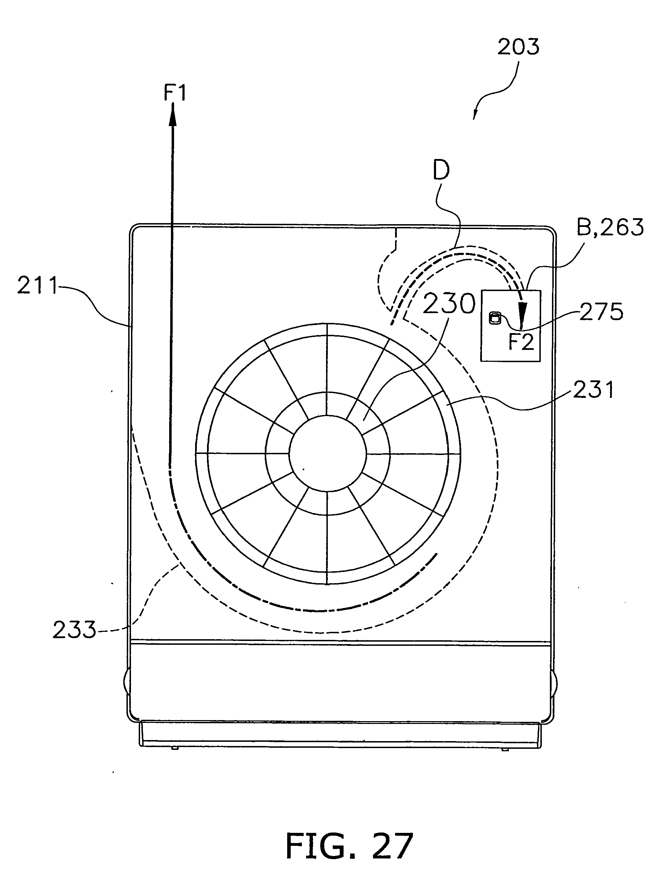

(A)
   In the first, second, third, and fourth embodiments, even though active species were generated using streamer discharge, active species may be generated by glow discharge, barrier discharge, or irradiating photocatalyst with ultraviolet light. In addition, if using glow discharge, discharging electrode may be coated with titanium apatite. Furthermore, if using barrier discharge, insulating material of discharge field region may be coated with titanium apatite.
(B)
   In the first embodiment, even though the blower 5 is provided inside the deodorizing agent casing 2, it may be provided outside the deodorizing agent casing 2. In addition, even though the deodorization function reproducing device of the first embodiment has a blower 5 combined therein, it may simply be a deodorization function reproducing device without the blower 5.
(C)
   In the first embodiment, even though the switch 7 becomes ON or OFF by opening or closing the door 2b, the switch 7 may be turned ON or OFF depending on if the deodorizing agent 4 is put in or taken out, and not limited to the opening and closing of the door 2b. Alternatively, another button switch or the like may be arranged to turn the switch 7 ON and OFF, or the switch 7 may be turned ON and OFF by a combination of these. In addition, even though the start and the end of the generation of active species are judged by the ON and OFF of the switch 7 through the opening and closing of the door 2b, the amount that the active species are generated may be increased or decreased by arranging a different adjustment switch, and turning the adjustment switch ON and OFF by opening or closing the door 2b.
(D)
   In the first embodiment, the blower 5 is disposed in the interior of the deodorizing agent casing 2, and an example of a case that airflow is generated in the interior of the deodorizing agent casing 2 was described.
   However, the present invention is not limited to this, for example, as shown in Fig. 26, the configuration may be to have a blower 105 disposed on the exterior of the deodorizing agent casing 2, and to have airflow generated inside the deodorizing agent casing 2 through an opening 20 and the like that is provided on the deodorizing agent casing 2. In addition, other configurations are same as that of the first embodiment, and the description thereof is omitted. In this case, the same effects as the above described embodiment can also be achieved.
(E)
   In the second embodiment, even though the switch 51 is turned ON and OFF by the opening and closing of the door 20b, the present invention is not limited to this, and the switch 51 may be turned ON and OFF depending on if the deodorizing agent 4a is put in or taken out. Alternatively, the switch 51 may be turned ON or OFF by providing another button switch or the like, or by a combination of these.
   In addition, even though the adjustment switch 52 is turned ON and OFF depending on if the deodorizing agent 4a is put in or taken out, the ON and OFF thereof may be controlled by the opening and closing of the door 20b. Alternatively, the ON and OFF thereof may be controlled by providing another button switch or the like, or through a combination of these.
(F)
   With the air purifier 200 of the above described fourth embodiment, an example of a case that active species are provided to the front side and rear side of the prefilter 241 was described.
   However, the present invention is not limited to this, for example, active species may only be provided to the rear side of the prefilter 241, or even by providing active species to the rear side of the plasma ion-wire 242, the effects can be obtained by the stable generation of the active species.
(G)
   With the air purifier 200 of the above described fourth embodiment, an example of a case that the deodorization catalytic cartridge 249 is disposed on a position along a portion of the bypass airflow path was described.
   However, the present invention is not limited to this, for example, a deodorization catalytic filter for adsorbing active species may be disposed and a bio-antibody filter may be disposed optionally on the downstream side thereof. By doing so, while the deodorization catalytic filter adsorbs the active species to decompose odor components, the bio-antibody filter is able to function with the bio-antibody put to work.
(H)
   With the air purifier 200 of the above described fourth embodiment, an example of a case that a portion of the air that is to flow out from near the blow out opening 212 becomes the bypass airflow F2 and is provided to the streamer discharging unit 263 through the openings of the unit storage portion B was described.
   However, the present invention is not limited to this, for example, separate from the pathway toward the blow out opening 212 from the blower fan 231, as shown in Fig. 27, the configuration may be to adopt an outward extending pathway D that can provide the flow of air from the blower fan 231 directly to the unit storage portion B and the streamer discharging unit 263.
(I)
   With the air purifier 200 of the above described fourth embodiment, an example of a case that one blower fan 231 being disposed on the most downstream side near the blow out opening 212 was described.
   However, the present invention is not limited to this, for example, it is acceptable as long as the disposition of the blower fan 231 allows both the main airflow F1 and the bypass airflow F2 of the above described embodiment to be formed. For example, the blower fan 231 may be disposed between any of the prefilter 241, plasma ion-wire 242, photocatalytic filter 243, and plasma catalytic filter 244 that make up the air purifying unit 240.
   In addition, the blower fan 231 may not just be one, but may have a configuration of two blower fans arranged, one for forming mainly the main airflow F1, and one for forming mainly the bypass airflow F2. In this case, the blower fan that generates the bypass airflow F2 may be disposed to force the bypass airflow F2 to be generated on the way of the bypass airflow path.

### INDUSTRIAL APPLICABILITY

The deodorization function reproducing device, deodorization function reproducing device and air conditioner in accordance with the present invention are useful as a reproducing device of deodorizing agent, a deodorization device including a reproducing device, an air conditioner including a deodorization function reproducing device, and the like, because odor and harmful gas components adsorbed to the deodorizing agent can be decomposed simply and reliably in a short period of time, without generating polluted water, and the deodorizing agent can be reproduced.

## Claims

1. A deodorization function reproducing device (1, 200) for performing a reproducing process of a deodorization functional agent (4, 249) to which at least one of odor molecules or harmful gas components floating in the air is adsorbed, the deodorization function reproducing device (1, 200) comprising:
a casing (2, B2) able to store the deodorization functional agent; and
an active species generating portion (3, 3a, 263) for generating active species that decompose or inactivate at least one of the odor molecules or harmful gas components.

2. The deodorization function reproducing device (1, 200) according to Claim 1, further comprising a blower (5, 230) for generating flow of air in the interior of the casing (2).

3. The deodorization function reproducing device (200) according to Claim 2, further comprising a purifying portion (240) for purifying air that passes through a position upwind of the storage portion of the deodorization functional agent and the active species generating portion (263), in the flow of the air that is generated by the blower (230).

4. The deodorization function reproducing device (1, 1a) according to one of any of Claims 1 to 3, wherein the active species generating portion (3, 3a, 263) generates the active species for a predetermined period of time.

5. The deodorization function reproducing device (1, 1a) according to Claim 4, wherein the active species generating portion (3, 3 a) can adjust the predetermined period of time.

6. The deodorization function reproducing device (1, 1a) according to Claim 4 or 5, further comprising a processing status detection means (6c to 6e) able to detect the processing status of the reproducing process,
wherein the active species generating portion (3, 3a) adjusts the predetermined period of time according to the processing status.

7. The deodorization function reproducing device (1, 1a, 200) according to Claim 6, further comprising a display portion (8, 53, 253) able to display the processing status.

8. The deodorization function reproducing device (1, 1a, 200) according to any of Claims 4 to 7, further comprising a switch (7, 51, B2S) able to judge the start and end of the reproducing process,
wherein the active species generating portion (3, 3a) starts generating the active species when the switch becomes ON and stops generating the active species when the switch becomes OFF.

9. The deodorization function reproducing device (1a) according to any of Claims 4 to 8, further comprising an adjustment switch (52) able to adjust the amount that the active species are generated,
wherein the active species generating portion (3, 3a) increases the amount that the active species are generated when the adjustment switch becomes ON and decreases the amount that the active species are generated when the adjustment switch becomes OFF.

10. The deodorization function reproducing device (1, 1a) according to Claim 8 or 9, wherein the switch (7, 51) or the adjustment switch (52) can be turned ON and OFF by the opening and closing a door (2b, 20b) of the casing (2, 20) from which the deodorization functional agent (4) can be put in or taken out.

11. The deodorization function reproducing device (la, 200) according to any of Claims 8 to 10, wherein the switch (7, 51, B2S) or the adjustment switch (52) can be turned ON and OFF depending on if the deodorization functional agent (4, 249) is put in or taken out, into or out of the casing (2, 20, B2).

12. The deodorization function reproducing device (1a) according to any of Claims 1 to 11, further comprising a deodorization functional agent (4) for adsorbing at least one of odor molecules or harmful gas components floating in the air,
wherein the deodorization function reproducing device (1a) forms an air conditioner.

13. An air conditioner (100), comprising:
a deodorization function reproducing device (1b) having a casing (67) able to store a deodorization functional agent, and an active species generating portion (3b) for generating active species that decompose or inactivate at least one of odor molecules or harmful gas components.
